# EUROPÄISCHE PATENTSCHRIFT

(11) **EP 2 833 959 B1**
(45) Veröffentlichungstag und Bekanntmachung des Hinweises auf die Patenterteilung: **05.03.2025**
(21) Anmeldenummer: 13715183.3
(22) Anmeldetag: 02.04.2013
(51) Int. Cl.: A61M 39/26, A61M 39/10

(54) **FLUIDVERBINDUNGSELEMENT**
FLUID CONNECTOR
CONNECTEUR POUR FLUIDE

(30) Priorität: 03.04.2012 DE 102012205490
(43) Veröffentlichungstag der Anmeldung: 11.02.2015
(73) Patentinhaber: Parker Hannifin Manufacturing Germany GmbH & Co. KG, 33659 Bielefeld (DE)
(72) Erfinder: AYDIN, Tolga, 74206 Bad Wimpfen (DE)
(74) Vertreter: Grauel, Andreas
(86) Internationale Anmeldenummer: PCT/EP2013/056959
(87) Internationale Veröffentlichungsnummer: WO 2013/150037

(56) Entgegenhaltungen:
- WO-A1-2011/100187
- WO-A1-2012/019164
- WO-A1-98/11931
- DE-A1- 3 023 583
- DE-U1- 202004 014 130
- DE-U1- 202004 014 130
- US-A- 4 421 296
- US-A- 5 163 922
- US-A1- 2010 176 162
- US-A1- 2011 276 035

## Beschreibung

### Technisches Gebiet

Die Erfindung betrifft ein Verbindungselement, insbesondere zum Verbinden von Gefäßen und zur Erzeugung einer Fluidverbindung zwischen an dem Verbindungselement angeschlossenen Gefäßen bzw. Elementen.

### Stand der Technik

Insbesondere im medizinischen Anwendungsbereich ist es im Stand der Technik bekannt, dass flüssige Lösungen oder Medikamente mittels Spritzen aus einem Vorratsgefäß aufgenommen werden und Patienten verabreicht werden. Dazu ist bekannt, dass mit der Spritze die Flüssigkeit direkt über die Kanüle der Spritze aufgenommen wird und anschließend direkt dem Patienten verabreicht wird.

Auch ist es bekannt, dass auf das Vorratsgefäß ein Verbindungselement aufgesetzt wird, woran dann die Spritze angebracht werden kann, um die Flüssigkeit aus dem Vorratsgefäß auf die Spritze aufzuziehen. Zieht man die Spritze anschließend wieder davon ab, so kann dennoch Flüssigkeit aus der Spritze auslaufen, was insbesondere bei sehr teuren Medikamenten sehr nachteilig ist. Auch ist damit eine Verschmutzung möglich, weil austretende Medikamente oder Flüssigkeiten verloren werden können, was im medizinischen Bereich oder im Pflegebereich als nachteilig angesehen ist.

Auch ist es bekannt, dass insbesondere Infusionslösungen mittels Schlauchleitungen mit einer Kanüle verbunden werden können, wobei auch in solche Schlauchleitungen oder Kanülen Medikamente zudosiert werden können. Dies erfolgt dann über ein Verbindungselement, das den gleichen Nachteilen unterliegt, wie bereits oben beschrieben. Derartige Verbindungselemente zur Erzeugung einer Fluidverbindung sind z.B. aus der US 2011/276035 A1, der DE 20 2004 014 130 U1 und der WO 98/11931 A1 bekannt.

### Darstellung der Erfindung, Aufgabe, Lösung, Vorteile

Es ist die Aufgabe der Erfindung, ein Verbindungselement zu schaffen, welches eine sichere Verbindung erlaubt und dennoch bei einem Nichtverbinden einen sicheren und dichten Abschluss des damit verbundenen Gefäßes erlaubt. Auch ist die Aufgabe der Erfindung ein elastisches Federelement zu schaffen, welches eine sichere Abdichtung bei gleichzeitiger einfacher Gestaltung erlaubt.

Dies wird erreicht mit den Merkmalen von Anspruch 1. Die abhängigen Ansprüche definieren zusätzliche Merkmale von bevorzugten Ausführungsbeispielen der Erfindung.

Im Folgenden werden einzelne Merkmale der Erfindung beschrieben. Dies betrifft zunächst ein Verbindungselement mit einem Gehäuse mit einem ersten Aufnahmeraum mit einem ersten Anschlusselement und einem darin angeordneten ersten verlagerbaren Ventilelement und mit einem zweiten Aufnahmeraum mit einem zweiten Anschlusselement mit einem darin angeordneten zweiten verlagerbaren Ventilelement, der erste und der zweite Aufnahmeraum sind miteinander fluidverbunden, wobei das erste Ventilelement entgegen der Kraft eines ersten Kraftspeichers verlagerbar ist und das zweite Ventilelement entgegen eines zweiten Kraftspeichers verlagerbar ist, wobei das erste Ventilelement zwischen zwei Positionen verlagerbar ist, wobei in der ersten Position eine Fluidverbindung zwischen dem ersten Aufnahmeraum und dem ersten Anschlusselement durch das erste Ventilelement unterbrochen ist und in der zweiten Position eine Fluidverbindung zwischen dem ersten Aufnahmeraum und dem ersten Anschlusselement durch das erste Ventilelement geöffnet ist und wobei das zweite Ventilelement zwischen zwei Positionen verlagerbar ist, wobei in der ersten Position eine Fluidverbindung zwischen dem zweiten Aufnahmeraum und dem zweiten Anschlusselement durch das zweite Ventilelement unterbrochen ist und in der zweiten Position eine Fluidverbindung zwischen dem zweiten Aufnahmeraum und dem zweiten Anschlusselement durch das zweite Ventilelement geöffnet ist.

Dies bewirkt, dass der erste Aufnahmeraum mit einem an das erste Anschlusselement anschließbaren Gefäß verbindbar ist und der zweite Aufnahmeraum mit einem an das zweite Anschlusselement anschließbaren Gefäß verbindbar ist, wobei im Falle eines Nichtanschließens an dem jeweiligen Anschlusselement, das Anschlusselement entsprechend mittels des diesbezüglich angeordneten Ventilelements verschlossen ist. Dadurch wird erreicht, dass das Verbindungselement dann einen Fluiddurchgang erzeugt, wenn an beiden Anschlusselementen ein Gefäß angeschlossen ist. Ist nur ein Gefäß angeschlossen, so ist das Anschlusselement verschlossen, an welchem kein Gefäß angeschlossen ist. Dies bewirkt, dass das Gefäß in diesem Betriebszustand auch mit angeordnetem Verbindungselement verschlossen ist.

Als Gefäß kann hier eine Spritze, eine Flasche, ein Schlauch, ein Beutel oder ähnliches verstanden werden, in welchem eine Flüssigkeit aufgenommen sein kann.

Desweiteren ist beschrieben ein Verbindungselement mit einem Gehäuse mit einem ersten Aufnahmeraum mit einem ersten Anschlusselement und mit einem zweiten Aufnahmeraum mit einem zweiten Anschlusselement mit einem darin angeordneten zweiten verlagerbaren Ventilelement, der erste und der zweite Aufnahmeraum sind miteinander fluidverbunden, wobei das zweite Ventilelement entgegen eines zweiten Kraftspeichers verlagerbar ist, wobei das zweite Ventilelement zwischen zwei Positionen verlagerbar ist, wobei in der ersten Position eine Fluidverbindung zwischen dem zweiten Aufnahmeraum und dem zweiten Anschlusselement durch das zweite Ventilelement unterbrochen ist und in der zweiten Position eine Fluidverbindung zwischen dem zweiten Aufnahmeraum und dem zweiten Anschlusselement durch das zweite Ventilelement geöffnet ist, wobei der erste Aufnahmeraum mit dem ersten Anschlusselement fluidverbunden ist.

Weiterhin ist beschrieben ein Verbindungselement mit einem Gehäuse mit einem ersten Aufnahmeraum mit einem ersten Anschlusselement und einem darin angeordneten ersten verlagerbaren Ventilelement und mit einem zweiten Aufnahmeraum mit einem zweiten Anschlusselement, der erste und der zweite Aufnahmeraum sind miteinander fluidverbunden, wobei das erste Ventilelement entgegen der Kraft eines ersten Kraftspeichers verlagerbar ist, wobei das erste Ventilelement zwischen zwei Positionen verlagerbar ist, wobei in der ersten Position eine Fluidverbindung zwischen dem ersten Aufnahmeraum und dem ersten Anschlusselement durch das erste Ventilelement unterbrochen ist und in der zweiten Position eine Fluidverbindung zwischen dem ersten Aufnahmeraum und dem ersten Anschlusselement durch das erste Ventilelement geöffnet ist, wobei der zweite Aufnahmeraum mit dem zweiten Anschlusselement fluidverbunden ist.

Dabei ist es bei einem Ausführungsbeispiel vorteilhaft, wenn das Gehäuse zumindest zweiteilig ausgebildet ist. Dabei sind die beiden Teile vorteilhaft miteinander verpresst, formschlüssig verbunden, verclipst, verschweißt, verklebt oder anderweitig verbunden. Dabei ist es insbesondere vorteilhaft, wenn die zwei Gehäuseteile vorteilhaft miteinander verbunden sind, wie insbesondere nicht zerstörungsfrei lösbar miteinander verbunden sind.

Auch ist es vorteilhaft, wenn das Gehäuse einteilig ausgebildet ist, wobei das Gehäuse aus mehreren Gehäuseteilen bestehen kann, die miteinander verbunden, wie insbesondere nicht zerstörungsfrei lösbar miteinander verbunden sind.

Bei einem Verpressen kann eine Verbindung ohne weitere Elemente kostengünstig erfolgen. Dabei stört auch kein Fremdmaterial die später durch das Verbindungselement fließenden Flüssigkeiten. Auch sorgt ein Kleben für eine zuverlässige dichte Verbindung.

Weiterhin ist es zweckmäßig, wenn der erste Aufnahmeraum von einem ersten und einem zweiten Gehäuseteil gebildet wird und der zweite Aufnahmeraum von dem zweiten Gehäuseteil gebildet wird. Dabei werden die beiden Gehäuseteile so miteinander verbunden, dass der Aufnahmeraum zwischen den beide Gehäusehälften entsteht. Dies kann vorteilhaft so erfolgen, dass die beiden Gehäuseteile einen Boden mit einer umlaufenden Wand aufweisen, die so ineinander gefügt werden, dass die beiden umlaufenden Wände aneinander anliegen und die beiden Böden beabstandet voneinander angeordnet sind und so zwischen sich ein Volumen für die Aufnahme oder Durchströmung einer Flüssigkeit erzeugen.

Vorteilhaft ist es, wenn das erste Anschlusselement an dem ersten Gehäuseteil angeordnet oder ausgebildet ist und das zweite Anschlusselement an dem zweiten Gehäuseteil angeordnet oder ausgebildet ist. Dabei ist es besonders vorteilhaft, wenn die beiden Anschlusselemente sich gegenüber liegen, so dass sich eine im Wesentlichen lineare Durchströmung ergibt. Dadurch kann eine günstige Geometrie erreicht werden und der Druckabfall bleibt bei gerader Durchströmung auch gering, was die Betätigungskräfte beispielsweise bei dem Aufziehen einer Spritze reduziert bzw. gering hält.

Es ist vorteilhaft, wenn der erste Aufnahmeraum einen im Schnitt etwa kreisförmigen Querschnitt aufweist und von einer umlaufenden Wand begrenzt ist, wobei der Aufnahmeraum von zwei etwa ebenen Wandbereichen begrenzt wird, wobei das erste Anschlusselement an einer der beiden Wände angeordnet oder aufgenommen ist und in der anderen Wand eine Fluidverbindung zu dem zweiten Aufnahmeraum vorgesehen ist. Der Aufnahmeraum ist vorteilhaft somit etwa zylindrisch mit einer umlaufenden Umfangswand und zwei sich etwa gegenüberliegenden Wänden. Dabei ist es vorteilhaft, wenn die Umfangswand eine Zylinderwand ist, die sich gegen eine entsprechende Wand des anderen Gehäuseteils abstützen kann, um eine gute Abdichtung zu erreichen.

Besonders vorteilhaft ist es, wenn das erste Anschlusselement ein hohlzylindrisches Element aufweist und das erste Ventilelement zumindest einen zylindrischen Bereich aufweist, welcher in dem hohlzylindrischen Element verlagerbar aufgenommen ist. Dabei ist es vorteilhaft, wenn das im Wesentlichen zylindrische Element aus einem elastischen Material gefertigt ist, so dass es sich an der Wand des hohlzylindrischen Elements anlegen und dort abdichten kann. Wird das Ventilelement durch Kraftbeaufschlagung verlagert, so wird eine Kante des zylindrischen Elements in oder über einen Bereich verschoben, in welchem beispielsweise eine Abweichung der hohlzylindrischen Kontur vorliegt oder eine Nut vorliegt, so dass eine Fluidverbindung zwischen dem Anschlusselement und dem Aufnahmeraum erzeugt wird.

Weiterhin ist es zweckmäßig, wenn das Ventilelement an dem zylindrischen Bereich abragende Federarme aufweist. Dabei kann der zylindrische Bereich an einer Seite Federarme aufweisen, vorteilhaft zumindest drei Federarme, die gleichmäßig zur Senkrechten abragen, wie beispielsweise die Kanten eines Tetraeders. Dabei ist es vorteilhaft, wenn die Federarme gleichmäßig verteilt abragen. Auch können mehr als drei Federarme, wie vier oder fünf oder mehr Federarme abragen.

Vorteilhaft ist es, wenn der zweite Aufnahmeraum einen Bereich mit im Schnitt kreisförmigen Querschnitt aufweist, der von einer umlaufenden Wand begrenzt ist, wobei der Aufnahmeraum von zwei etwa ebenen Wandbereichen begrenzt wird, wobei das zweite Anschlusselement an einer der beiden Wände angeordnet oder aufgenommen ist und in der anderen Wand eine Fluidverbindung zu dem ersten Aufnahmeraum vorgesehen ist. Die Wand zur Erzeugung einer Fluidverbindung zum ersten Aufnahmeraum wird dabei vorteilhaft durch das gleiche Element gebildet, welches auch die Wand des ersten Aufnahmeraums bildet, welche die Fluidverbindung mit dem zweiten Aufnahmeraum erzeugt. Dabei ist es vorteilhaft, wenn die jeweilige Wand sich an einem Element befindet und die jeweils gegenüberliegende Wand einer Platte, Scheibe oder ähnliches ist. Die Fluidverbindung wird dann vorteilhaft durch zumindest eine, vorteilhaft eine Mehrzahl von Öffnungen bewirkt.

Auch ist es vorteilhaft, wenn der erste Aufnahmeraum einen im Schnitt kreisförmigen Querschnitt mit nach außen ragenden Taschen aufweist und von einer im Radius veränderlichen umlaufenden Wand begrenzt ist, wobei der Aufnahmeraum von zwei etwa ebenen Wandbereichen begrenzt wird, wobei das erste Anschlusselement an einer der beiden Wände angeordnet oder aufgenommen ist und in der anderen Wand eine Fluidverbindung zu dem zweiten Aufnahmeraum vorgesehen ist. Durch die Ausbildung der umlaufenden Taschen wird das Volumen des Aufnahmeraums reduziert, ohne die Funktion des Aufnahmeraums zu beeinträchtigen. Dies ist insbesondere bei teuren Flüssigkeiten vorteilhaft, weil die im Aufnahmeraum verbleibende Flüssigkeit verloren geht.

Besonders vorteilhaft ist es, wenn die Anzahl der Taschen der Anzahl von Federarmen des Ventilelements entspricht. Dies ist daher vorteilhaft, weil die Federarme sicher aufgenommen werden können und kein weiteres Totvolumen geschaffen wird.

Besonders vorteilhaft ist es, wenn in jeder Tasche ein Federarm des Ventilelements angeordnet ist.

Weiterhin ist es vorteilhaft, wenn von der anderen Wand ein Dorn abragt, welcher in das zweite Anschlusselement eingreift. Der Dorn ist vorteilhaft ein flacher oder zylindrischer Steg, der in eine Hülse des zweiten Anschlusselements eingreift. Dabei hat der Dorn vorteilhaft an seinem Ende einen verdickten Bereich, an welchem sich die Hülse endseitig abstützt.

Auch ist es vorteilhaft, wenn die eine Wand mit dem Anschlusselement als zweites Ventilelement entgegen der Kraft eines Kraftspeichers verlagerbar ist und dadurch eine Fluidverbindung zwischen dem Anschlusselement und dem in das Anschlusselement hineinragenden Dorn freigebbar ist.

Vorteilhaft ist es weiterhin, wenn der zweite Kraftspeicher ein elastisches Element ist, welches in dem zweiten Aufnahmeraum angeordnet ist.

Dabei ist es zweckmäßig, wenn der zweite Kraftspeicher ein elastischer Ring ist, wie vorzugsweise ein elastischer Ovalring oder O-Ring.

Erfindungsgemäß ist es vorteilhaft, wenn die von dem zylindrischen Bereich bzw. von dem zylindrischen Körper abragenden, als gerade oder einfach oder mehrfach abgeknickte oder gekrümmte Federarme ausgebildet sind. Durch das Abknicken kann eine bessere Aufstandsfläche erreicht werden und die Arme dienen besser der Funktion den zylindrischen Bereich bzw. Körper in vertikaler Richtung entgegen der elastischen Federkraft der Federarme verlagern zu lassen.

Besonders vorteilhaft ist es, wenn die Federarme des ersten Ventilelements in einem Winkel von 120° zueinander in der Ebene senkrecht zur Längsachse des zylindrischen Bereichs angeordnet sind. Dazu ist es vorteilhaft, wenn drei Federarme vorgesehen sind.

Auch ist es bei einem Ausführungsbeispiel vorteilhaft, wenn die Federarme einen ersten Bereich aufweisen, welcher in einem Winkel von etwa 25 bis 30°, vorzugsweise 27°, zur Ebene senkrecht zur Längsachse des zylindrischen Bereichs angeordnet ist. Dieser Bereich schließt sich vorteilhaft direkt an den zylindrischen Bereich an.

Auch ist es vorteilhaft, wenn die Federarme einen zweiten Bereich aufweisen, welcher in einem Winkel von etwa 35 bis 40°, vorzugsweise 37°, zur Ebene senkrecht zur Längsachse des zylindrischen Bereichs angeordnet ist. Dieser folgt dem ersten Bereich nach.

Auch ist es zweckmäßig, wenn die Federarme einen dritten Bereich aufweisen, welcher in einem Winkel von etwa 75 bis 80°, vorzugsweise 77°, zur Ebene senkrecht zur Längsachse des zylindrischen Bereichs angeordnet ist. Dieser Bereich folgt dem zweiten Bereich und bildet den Bereich, der auf einer Wandung, wie dem Boden, des Aufnahmeraums aufsteht.

Weiterhin beschrieben wird ein elastisches Federelement mit einem zylindrischen Körper und davon abragenden Federarmen, wobei das Federelement aus einem elastischen Material gefertigt ist. Dabei ragen insbesondere drei Federarme von dem zylindrischen Körper ab, wobei die vorzugsweise drei Federarme in einem Winkel von 120° in der Ebene senkrecht zur Mittelachse des zylindrischen Körpers verteilt angeordnet sind.

Es ist vorteilhaft, wenn die abragenden Federarme als gerade oder einfach oder mehrfach abgeknickte oder gekrümmte Federarme ausgebildet sind. Durch das Abknicken oder das Krümmen kann der Federarm besser abgestützt werden und dennoch eine günstige Federcharakteristik für die Federung des zylindrischen Bereichs bewirken.

Dabei ist es vorteilhaft, wenn die Federarme des ersten Ventilelements in einem Winkel von 120° zueinander in der Ebene senkrecht zur Längsachse des zylindrischen Bereichs angeordnet sind. Dabei ist es besonders vorteilhaft, wenn drei Federarme vorgesehen sind.

Besonders vorteilhaft ist es, wenn die Federarme einen ersten Bereich aufweisen, welcher in einem Winkel von etwa 25 bis 30°, vorzugsweise 27°, zur Ebene senkrecht zur Längsachse des zylindrischen Bereichs angeordnet ist.

Auch ist es vorteilhaft, wenn die Federarme einen zweiten Bereich aufweisen, welcher in einem Winkel von etwa 35 bis 40°, vorzugsweise 37°, zur Ebene senkrecht zur Längsachse des zylindrischen Bereichs angeordnet ist.

Ebenso ist es vorteilhaft, wenn die Federarme einen dritten Bereich aufweisen, welcher in einem Winkel von etwa 75 bis 80°, vorzugsweise 77°, zur Ebene senkrecht zur Längsachse des zylindrischen Bereichs angeordnet ist.

Es ist weiterhin vorteilhaft, wenn der Querschnitt eines Federarms rund oder oval oder mehreckig ausgebildet ist.

Dabei ist es besonders vorteilhaft, wenn der Querschnitt des Federarms oval ist, wobei der Querschnitt in einer Ebene durch die Mittelsenkrechte des Federelements einen kleineren Durchmesser aufweist als die dazu senkrechte Richtung. Dadurch lässt sich ein günstiges Biegeverhalten der Federarme erzielen.

Auch ist es zweckmäßig, wenn der zylindrische Körper an einem Endbereich benachbart zu dem Übergang zu den Federarmen eine erste umlaufende Dichtlippe aufweist.

Weiterhin ist es zweckmäßig, wenn der zylindrische Körper an einem Endbereich gegenüber dem Übergang zu den Federarmen eine zweite umlaufende Dichtlippe aufweist. Dadurch lässt sich die Dichtwirkung verbessern.

Auch ist es zweckmäßig, wenn die erste Dichtlippe als umlaufender Absatz ausgebildet ist. Dabei geht der Absatz von einem Durchmesserbereich größeren Durchmessers zu einem Durchmesserbereich kleineren Durchmessers über.

Besonders vorteilhaft ist es, wenn die zweite Dichtlippe als von der zylindrischen Wandung des zylindrischen Körpers vorragende Lippe ausgebildet ist. Die Lippe steht dabei in radialer Richtung von dem zylindrischen Körper ab.

Weitere vorteilhafte Ausgestaltungen sind durch die nachfolgende Figurenbeschreibung und durch die Unteransprüche beschrieben.

### Kurze Beschreibung der Zeichnungen

Nachstehend wird die Erfindung auf der Grundlage zumindest eines Ausführungsbeispiels anhand der Zeichnungen näher erläutert. Es zeigen:
- Fig. 1: einen Schnitt eines ersten Ausführungsbeispiels eines erfindungsgemäßen Verbindungselements,
- Fig. 2: einen weiteren Schnitt durch das Verbindungselement, und
- Fig. 3: eine Ansicht von oben auf das Verbindungselement,
- Fig. 4: eine perspektivische Darstellung des Verbindungselements,
- Fig. 5: eine Explosionsdarstellung eines Verbindungselements,
- Fig. 6: einen Schnitt eines weiteren Ausführungsbeispiels eines erfindungsgemäßen Verbindungselements,
- Fig. 7: eine Seitenansicht eines Verbindungselements gemäß Figur 6,
- Fig. 8: eine Ansicht von oben auf das Verbindungselement gemäß Figur 5 oder 6,
- Fig. 9: einen Schnitt eines weiteren Ausführungsbeispiels eines erfindungsgemäßen Verbindungselements,
- Fig. 10: eine Seitenansicht eines Verbindungselements gemäß Figur 9,
- Fig. 11: eine Ansicht von oben auf das Verbindungselement gemäß Figur 9 oder 10,
- Fig. 12: eine Explosionsdarstellung des Verbindungselements gemäß Figur 9,
- Fig. 13: einen Schnitt eines weiteren Ausführungsbeispiels eines erfindungsgemäßen Verbindungselements,
- Fig. 14: eine Explosionsdarstellung des Verbindungselements gemäß Figur 13,
- Fig. 15: einen Schnitt eines weiteren Ausführungsbeispiels eines erfindungsgemäßen Verbindungselements,
- Fig. 16: eine Explosionsdarstellung des Verbindungselements gemäß Figur 13,
- Fig. 17: eine Darstellung eines Details des Verbindungselements nach Figur 9 im geschlossenen Zustand,
- Fig. 18: eine Darstellung eines Details des Verbindungselements nach Figur 9 im geöffneten Zustand,
- Fig. 19: eine Darstellung eines Details des Verbindungselements nach Figur 6,
- Fig. 20: eine Darstellung eines Details des Verbindungselements nach Figur 9,
- Fig. 21: eine Ansicht des elastischen Federelements von oben,
- Fig. 22: ein Schnitt des elastischen Federelements entlang Linie A-A der Figur 21,
- Fig. 23: ein Schnitt des elastischen Federelements entlang Linie B-B der Figur 22, und
- Fig. 24: eine vergrößerte Detailansicht des oberen Bereichs des elastischen Federelements gemäß Figur 22.

### Bevorzugte Ausführung der Erfindung

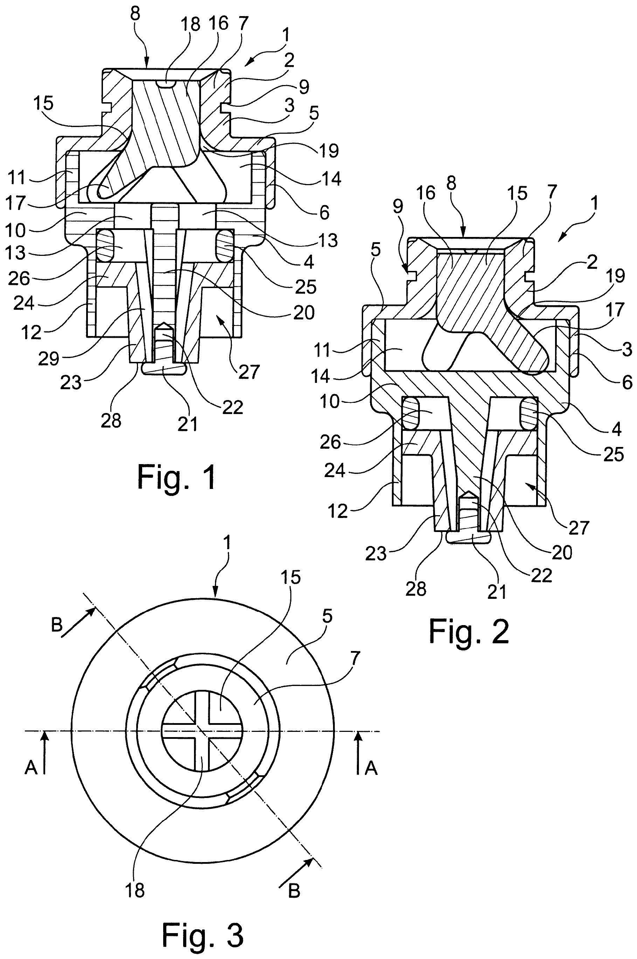

Die Figuren 1 und 2 zeigen ein Ausführungsbeispiel eines erfindungsgemäßen Verbindungselements 1 jeweils im Schnitt gemäß den Linien A-A und B-B der Figur 3. Dabei zeigt Figur 1 den Schnitt gemäß Linie B-B und die Figur 2 den Schnitt gemäß Linie A-A.

Das Verbindungselement 1 ist mit einem Gehäuse 2 ausgebildet. Das Gehäuse 2 besteht aus zumindest zwei Gehäuseteilen 3,4. Im Ausführungsbeispiel der Figur 1 besteht das Gehäuse 2 aus den beiden Gehäuseteilen 3,4, die miteinander verbunden sind. Bei weiteren Ausführungsbeispielen kann das Gehäuse auch einteilig oder mehrteilig mit mehr als drei Gehäuseteilen ausgebildet sein.

Das erste Gehäuseteil 3 weist einen Boden 5 mit einer daran im Winkel von 90° abragenden umlaufenden Wand 6 auf. Der Boden 5 ist mit dem Anschlusselement 7 versehen. Dazu ist das Anschlusselement 7 als Anschlussstutzen an dem Boden 5 angebracht. Vorteilhaft ist es mit dem Boden einteilig verbunden. Dabei kann in die Öffnung 8 des Anschlusselements 7 ein zu verbindendes Element eingesteckt werden. Zur Fixierung ist radial außerhalb des Anschlussstutzens in der Wand des Stutzens ein Gewinde 9 vorgesehen, mittels welchem das zu verbindende Element auch mit dem Anschlussstutzen verschraubt werden kann.

Das zweite Gehäuseteil 4 ist mit einem Boden 10 ausgebildet, von dem sich in eine Richtung eine nach oben abragende umlaufende Wand 11 erstreckt. In die entgegengesetzte Richtung ist eine umlaufende Wand 12 vorgesehen, die ebenso von dem Boden 10 abragt. Der Boden 10 weist Öffnungen 13 auf, um eine Fluidkommunikation zwischen den beiden Seiten des Bodens 10 zu erlauben. Dabei ist im Ausführungsbeispiel eine Mehrzahl von Öffnungen vorgesehen, wobei auch nur eine Öffnung vorgesehen sein könnte. Vorzugsweise sind vier Öffnungen 13 vorgesehen. Vorteilhaft ist eine Anzahl von vier Öffnungen 13 vorgesehen, die durch einen kreuzförmigen Steg getrennt sind.

Wie in den Figuren 1 und 2 zu erkennen ist, sind die beiden Gehäuseteile 3,4 im Bereich der umlaufenden Wände 6,11 ineinander gesteckt und vorzugsweise miteinander verpresst. Die Wände 6, 11 sind dabei derart ausgebildet, dass die axiale Ausdehnung der Wände 6, 11 etwa gleich lang ist und der Innenradius der Wand 6 etwa dem Außenradius der Wand 11 entspricht, so dass sie ineinander gesteckt werden können und miteinander verpresst werden können.

Alternativ kann auch die Wand 6 des ersten Gehäuseteils 3 radial innen von der Wand 11 des zweiten Gehäuseteils 4 angeordnet sein.

Zur Verbindung der beiden Gehäuseteile 3,4 im Bereich der Wände 6,11 kann ein Verpressen vorteilhaft sein. Auch kann ein Verkleben, Verschweißen, Verclipsen etc. anwendbar sein.

Das Gehäuse bildet einen ersten Aufnahmeraum 14 im Bereich der Verbindung der beiden Gehäuseteile 3,4, wobei der Aufnahmeraum 14 gebildet wird durch eine umlaufende Wand 11, einen Boden 5 des ersten Gehäuseteils und einen Boden 10 des zweiten Gehäuseteils.

Nach oben am Boden 5 des ersten Gehäuseteils 3 ist das Anschlusselement vorgesehen, wie bereits oben beschrieben. Wird ein mit dem Anschlusselement 7 zu verbindendes Element, wie beispielsweise eine Spritze oder ähnliches, bzw. ein daran angebrachter Stutzen in das Anschlusselement 7 eingesteckt, so soll eine Fluidkommunikation zwischen dem zu verbindenden Element und dem Aufnahmeraum 14 erfolgen können. Wird kein zu verbindendes Element in das Anschlusselement eingesteckt, so soll das Anschlusselement 7 abgeschlossen und abgedichtet sein.

Dies wird erreicht mit einem Ventilelement 15. Das Ventilelement 15 weist einen zylindrischen Bereich 16 auf, von dem nach unten Federarme 17 abragen. Dabei ist der zylindrische Bereich mit den Federarmen vorteilhaft einteilig ausgebildet und die Federarme ragen in einem Winkel von etwa 45° zur Vertikalen nach radial außen und unten von dem zylindrischen Bereich 16 ab.

Die Federarme 17 bilden bei Vorhandensein von drei Federarmen eine Anordnung gemäß einer Kantenanordnung eines Tetraeders. Am oberen Ende des zylindrischen Bereichs 16 ist eine kreuzförmige Nut 18 eingebracht, welche dazu dient, eine Fluidverbindung zwischen dem Anschlusselement 7 und dem Aufnahmeraum 14 zu gewährleisten.

In den Figuren 1 und 2 gezeigten Positionen des Ventilelements 15 ist eine Fluidverbindung zwischen dem Anschlusselement 7 und dem Aufnahmeraum 14 abgeschlossen und abgedichtet. Wird von oben in das Anschlusselement 7 ein Element eingesteckt, so wird der zylindrische Bereich 16 des Ventilelements 15 komprimiert und nach unten geschoben, so dass sich die Federarme 17 in den Ecken des Aufnahmeraums 14 abstützen und elastisch verformen. Dies wird soweit erfolgen, bis eine Fluidverbindung zwischen dem kreuzförmigen Kanal 18 und einer Abrundung 19 im Bereich des Anschlusselementes vorliegt. Dann kann eine Fluidverbindung zwischen dem zu verbindenden Element und dem Aufnahmeraum 14 erfolgen. Statt der Abrundung können auch Nuten oder Kanäle in der Wandung des Anschlusselements vorgesehen sein, welche ab einer gewissen vorgebbaren Position des Ventilelements eine Fluidverbindung erlauben.

Wird das zu verbindende Element wieder aus dem Anschlusselement 7 entfernt, so entspannt sich das elastische formbare Ventilelement 15 wieder und der zylindrische Bereich 16 wird wieder nach oben geschoben, so dass das Anschlusselement 7 wieder abgedichtet ist.

Im unteren Bereich des Verbindungselements 1 ist radial innerhalb des zylindrischen Wandbereichs 12 ein Dorn 20 vorgesehen, welcher an seinem Ende ein verdicktes Element 21 trägt, dass bevorzugt in eine Öffnung 22 des Dorns 20 eingesteckt ist oder bevorzugt einteilig mit dem Dorn 20 ausgebildet ist. Der Dorn 20 kann vorzugsweise mit einem Steg einteilig ausgebildet sein, welcher die Öffnungen 13 trennt.

Als Ventilelement ist ein hülsenförmiges Element 23 vorgesehen, welches in einen Boden 24 übergeht, wobei das hülsenförmige Element 23 etwa hohlzylindrisch ausgebildet ist und sich mit seinem axial unteren Randbereich an einer Schulter des Elements 21 abstützt. Zwischen dem Boden 24 und dem Boden 10 des Gehäuseteils ist ein Kraftspeicher 25, bevorzugt als ringförmiges elastisches Element, angeordnet, um eine Beaufschlagung des hülsenförmigen Elements 23 gegen das Element 21 zu beaufschlagen. Dadurch wird eine Abdichtung zwischen dem Aufnahmeraum 26 und dem Anschlusselement 27 erreicht, das durch das hülsenförmige Element 23 mit dem Boden 24 und der Umfangswandung 12 gebildet ist. Das hülsenförmige Element 23 ist bevorzugt zylindrisch oder zumindest etwas konisch ausgebildet, so dass die obere Öffnung größer ist als die untere Öffnung, die sich an dem Element 21 abstützt.

Wird von unten ein zu verbindendes Element mit einem umlaufenden Kragen in den Bereich 27 eingesteckt, so wird das hülsenförmige Element 23 mit dem Boden 24 axial nach oben geschoben, entgegen der Kraft des Kraftspeichers 25 und die Schulter 28 des hülsenförmigen Elements 23 hebt von einer Schulter des Elements 21 ab und erlaubt eine Fluidverbindung zwischen dem zu verbindenden Element durch den Kanal 29 im hülsenförmigen Element 23 bis zum Aufnahmeraum 26. Da zwischen dem Aufnahmeraum 26 und dem Aufnahmeraum 14 durch die Öffnungen 13 eine Fluidverbindung besteht, kann zwischen zwei zu verbindenden Elementen, die an den Anschlusselementen 7 und 27 angeschlossen sind, eine Fluidverbindung erzeugt werden.

Bevorzugt sind die elastischen Elemente 15 und 25 aus einem elastomeren Material und die Gehäuseelemente 3, 4 sowie das hülsenförmige Element 23 und das Element 21 aus einem nicht elastomeren, eher formstabilen, Material, wie aus einem Thermoplast. Dabei kann das Element 15 und das Element 25 bevorzugt aus Liquid Silicone Rubber (LSR) oder Hochtemperatur vernetzendem Silikonkautschuk (HTV) oder Raumtemperatur vernetzendem Silikonkautschuk (RTV) bestehen und die Gehäuseteile sowie die Hülse und das Abschlusselement 21 können aus Acrylnitril/Butadien/Styrol (ABS), Polycarbonat (PC), Polypropylen (PP) oder Polyethylen (PE) o.ä. bestehen.

Das Element 21 kann, wie gezeigt, in eine Öffnung des Dorns 20 eingesteckt sein. Alternativ kann das Element 21 auch durch Heißverpressen des Dorns durch eine diesbezügliche Verformung des Dorns entstehen. Gegebenenfalls kann es auch an den Dorn angespritzt sein.

Vorteilhaft kann auch sein, wenn zwischen dem Element 21 und dem hülsenförmigen Element 23 ein elastomeres Material im Bereich der Schulter des Elements 21 vorgesehen ist, wie beispielsweise eine Scheibe die beispielsweise auch als anvulkanisiertes Material vorgesehen sein kann, um eine bessere Abdichtung zwischen dem Element 21 und Element 23 zu bewirken.

Alternativ zur Ausbildung des Kraftspeichers 25 als elastomerer Ring kann dieser beispielsweise als Ovalring oder O-Ring ausgebildet sein oder auch als anders ausgebildeter Kraftspeicher, wie beispielsweise ein Federkraftspeicher. Auch die Federarme 17 des zylindrischen Elements 15 können alternativ auch anderweitig ausgebildet sein, wie beispielsweise durch einen zentralen elastischen Fortsatz, der in axialer Richtung das Element 15 verlängert. Dabei kann der Fortsatz auch zusätzlich zu den Federarmen vorgesehen sein. Vorteilhaft könnte es auch sein, wenn das bislang als unsymmetrisch ausgebildete Element durch Vorsehung zweier identischer oder im Wesentlichen identischer Bauteile oder Bereiche symmetrisch gestaltet ist, so dass beispielsweise das Ventilelement 15 beiderseits des Bodens 10 entsprechend angeordnet ist, um zwei beiderseits des Bodens 10 angeordnete Aufnahmeräume mit gleichen Ventilelementen zu verschließen. Entsprechend könnte auch die alternative Anordnung zweier Ventilelemente gemäß 23, 24 vorgesehen sein, die beiderseits der Wandung 10 angeordnet sind. Dadurch könnte eine auf beiden Seiten der Wandung 10 angeordnete identische Ventileinheit vorgesehen sein, die durch eine Gleichheit der Bauteile auch einfach herzustellen wäre.

Die Figur 3 zeigt das Verbindungselement 1 in einer Ansicht von oben, bei der der Boden 5 als scheibenförmiger Boden zu erkennen ist, von welchem das Anschlusselement 7 kreisringförmig nach oben abragt. In der zentralen Öffnung des kreisringförmigen Anschlusselements 7 ist das Ventilelement 15 mit der kreuzförmigen Fluidverbindungsnut 18 zu erkennen.

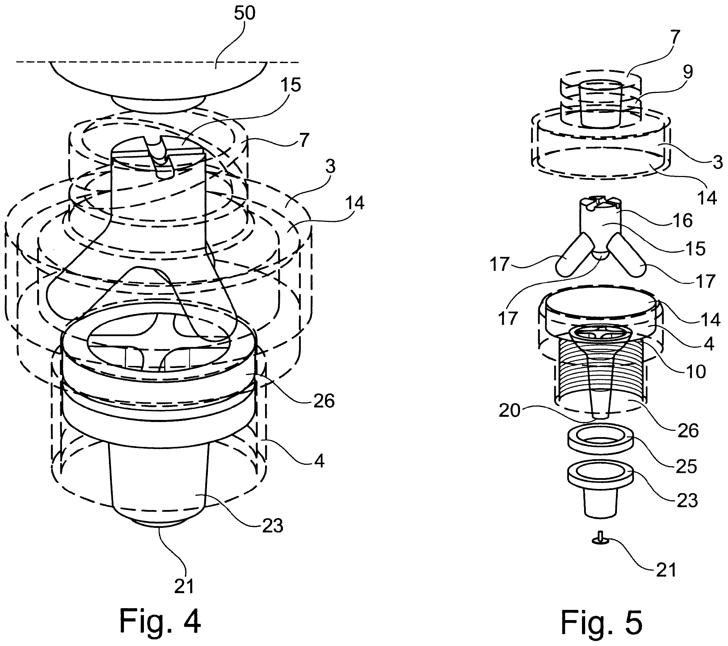

Die Figur 4 zeigt das erfindungsgemäße Verbindungselement 1 in einer dreidimensionalen schematischen Darstellung und die Figur 5 zeigt das Verbindungselement 1 in einer Explosionsdarstellung.

Wie zu erkennen ist, bilden die Gehäuseteile 3, 4 das Gehäuse zur Aufnahme der Ventilelemente 15, 23, wobei diese die Aufnahmeräume 14, 26 abschließen können.

In Figur 5 ist zuoberst das Gehäuseteil 3 zu erkennen, das einen wesentlichen zylindrischen Raumbereich 14 definiert und an seinem oberen Ende das Anschlusselement 7 als Anschlussstutzen aufweist. Am äußeren Rand beziehungsweise an der äußeren Wandung des Anschlusselements 7 ist ein Gewinde 9 vorgesehen zur Verschraubung mit einem zu verbindenden Element. Dieses zu verbindenden Element ist in Figur 4 schematisch als das Element 50 dargestellt.

Das Ventilelement 15 ist mit einem zylindrischen Körper 16 und Federarmen 17 ausgebildet, die schräg zur Senkrechten abstehen. Dabei sind die Federarme 17 im Ausführungsbeispiel der Figur 5 ähnlich von Kanten eines Tetraeders ausgerichtet. Das Gehäuse 4 weist einen Aufnahmeraum auf, der zusammen mit dem Aufnahmeraum 14 des Gehäuseteils 3 den Aufnahmeraum bilden. Unterhalb der als Boden bezeichneten Wandung 10 ist ein weiterer Aufnahmeraum 26 definiert, in welchem das elastische Element 25 und das hülsenförmige Element 23 eingesetzt werden bevor der verdickte Abschluss 21 des Dorns 20 eingefügt wird.

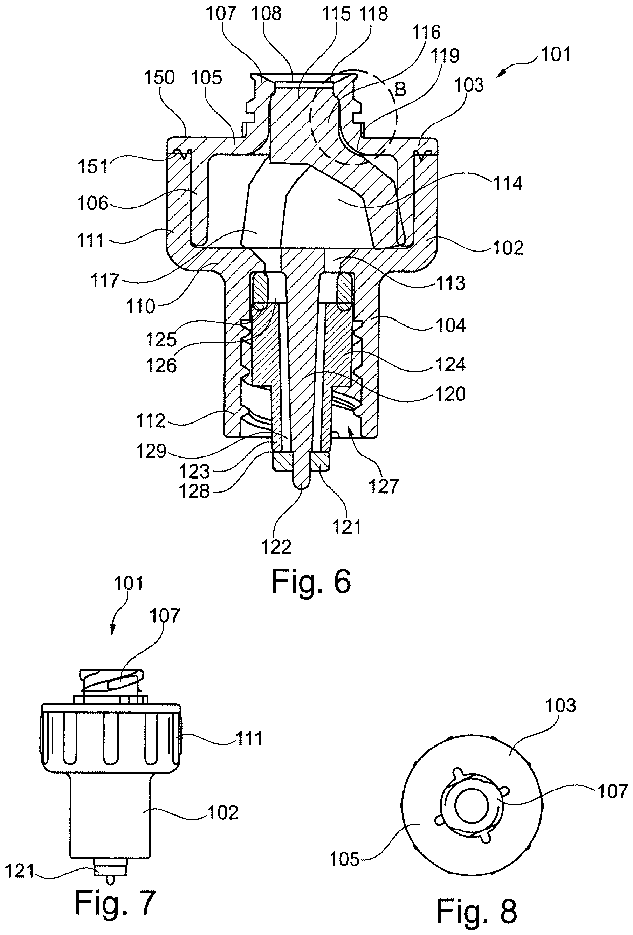

Die Figuren 6, 7 und 8 zeigen ein weiteres Ausführungsbeispiel eines erfindungsgemäßen Verbindungselements 101 jeweils im Schnitt bzw. in einer Seitenansicht bzw. in einer Ansicht von oben.

Das Verbindungselement 101 ist mit einem Gehäuse 102 ausgebildet. Das Gehäuse 102 besteht aus zumindest zwei Gehäuseteilen 103,104. Im Ausführungsbeispiel der Figur 6 besteht das Gehäuse 102 aus den beiden Gehäuseteilen 103, 104, die miteinander verbunden sind.

Das erste Gehäuseteil 103 weist einen Boden 105 mit einer daran im Winkel von 90° abragenden umlaufenden Wand 106 auf. Der Boden 105 ist mit dem Anschlusselement 107 versehen. Dazu ist das Anschlusselement 107 als Anschlussstutzen an dem Boden 105 angebracht. Vorteilhaft ist es mit dem Boden einteilig verbunden. Dabei kann in die Öffnung 108 des Anschlusselements 107 ein zu verbindendes Element eingesteckt werden. Zur Fixierung ist radial außerhalb des Anschlussstutzens 107 in der Wand des Stutzens ein Gewinde 109 oder ähnliches vorgesehen, mittels welchem das zu verbindende Element auch mit dem Anschlussstutzen 107 verschraubt oder verbunden werden kann.

Das zweite Gehäuseteil 104 ist mit einem Boden 110 ausgebildet, von dem sich in eine Richtung eine nach oben abragende umlaufende Wand 111 erstreckt. In die entgegengesetzte Richtung ist eine umlaufende Wand 112 vorgesehen, die ebenso von dem Boden 110 abragt. Der Boden 110 weist zumindest eine Öffnung oder mehrere Öffnungen 113 auf, um eine Fluidkommunikation zwischen den beiden Seiten des Bodens 110 zu erlauben. Dabei ist im Ausführungsbeispiel eine Mehrzahl von Öffnungen 113 vorgesehen, wobei auch nur eine Öffnung vorgesehen sein könnte. Vorzugsweise sind vier Öffnungen 113 vorgesehen. Vorteilhaft ist eine Anzahl von vier Öffnungen 113 vorgesehen, die durch einen kreuzförmigen Steg getrennt sind.

Wie in der Figur 6 zu erkennen ist, sind die beiden Gehäuseteile 103,104 im Bereich der umlaufenden Wände 106,111 ineinander gesteckt und vorzugsweise miteinander verpresst oder verklebt. Die Wände 106, 111 sind dabei derart ausgebildet, dass die axiale Ausdehnung der Wände 106, 111 etwa gleich lang ist und der Innenradius der Wand 111 etwa dem Außenradius der Wand 106 entspricht, so dass sie ineinander gesteckt werden können und miteinander verpresst oder verklebt oder verschweißt werden können. Die Wand 106 ist dabei radial innerhalb der Wand 111.

Zur Verbindung der beiden Gehäuseteile 3,4 im Bereich der Wände 6,11 kann ein Verpressen vorteilhaft sein. Auch kann ein Verkleben, Verschweißen, Verclipsen etc. anwendbar sein. Auch kann die Verbindung zwischen dem Flansch 150 und der Oberseite 151 der Wandung 111 erfolgen. Dies kann auch durch Schweißen erfolgen.

Das Gehäuse bildet einen ersten Aufnahmeraum 114 im Bereich der Verbindung der beiden Gehäuseteile 103,104, wobei der Aufnahmeraum 114 gebildet wird durch eine umlaufende Wand 106, einen Boden 105 des ersten Gehäuseteils und einen Boden 110 des zweiten Gehäuseteils.

Nach oben am Boden 105 des ersten Gehäuseteils 103 ist das Anschlusselement 107 vorgesehen. Wird ein mit dem Anschlusselement 107 zu verbindendes Element, wie beispielsweise eine Spritze oder ähnliches, bzw. ein daran angebrachter Stutzen in das Anschlusselement 107 eingesteckt, so soll eine Fluidkommunikation zwischen dem zu verbindenden Element und dem Aufnahmeraum 114 erfolgen können. Wird kein zu verbindendes Element in das Anschlusselement 107 eingesteckt, so soll das Anschlusselement 107 abgeschlossen und abgedichtet sein.

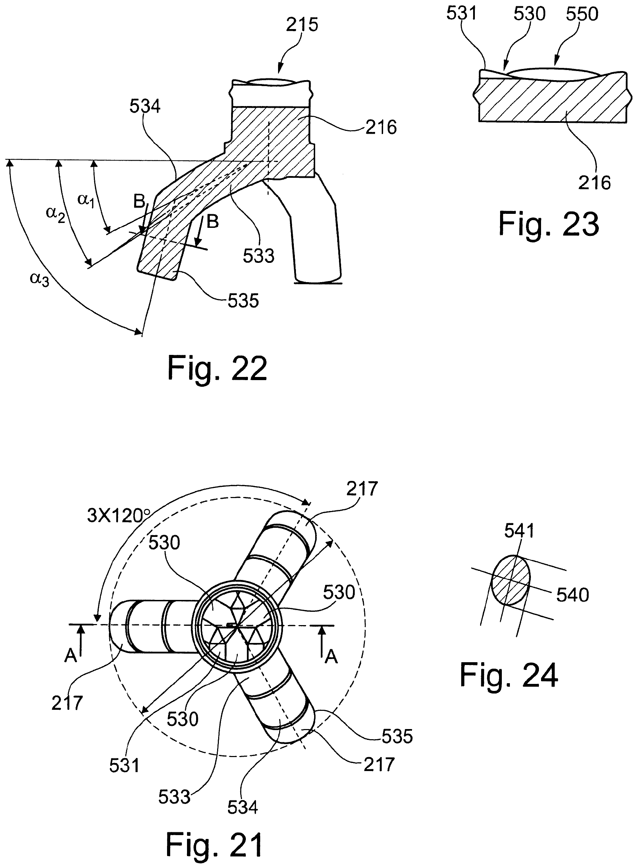

Dies wird erreicht mit einem Ventilelement 115. Das Ventilelement 115 weist einen zylindrischen Bereich 116 oder auch einen zylindrischen Körper 116 auf, von dem nach unten Federarme 117 abragen. Dabei ist der zylindrische Bereich 116 mit den Federarmen 117 vorteilhaft einteilig ausgebildet und die Federarme 117 ragen in einem Winkel zur Vertikalen nach radial außen und unten von dem zylindrischen Bereich 116 ab. Das elastische Federelement wird in den Figuren 21 bis 24 näher erläutert. Die Federarme sind in dem Ausführungsbeispiel der Figuren 6 bis 8 abgewinkelt bzw. weisen Knicke auf. Die Federarme 117 bilden bei Vorhandensein von drei Federarmen 117 eine Anordnung annähernd einer Kantenanordnung eines Tetraeders.

Am oberen Ende des zylindrischen Bereichs 116 ist eine dreieckförmige Nut 118 eingebracht, welche dazu dient, eine Fluidverbindung zwischen dem Anschlusselement 107 und dem Aufnahmeraum 114 zu gewährleisten, wenn das zylindrische Element 116 nach unten gedrückt wird.

In der in Figur 6 gezeigten Position des Ventilelements 115 ist eine Fluidverbindung zwischen dem Anschlusselement 107 und dem Aufnahmeraum 114 abgeschlossen und abgedichtet. Wird von oben in das Anschlusselement 107 ein Element eingesteckt, so wird der zylindrische Bereich 116 des Ventilelements 115 nach unten beaufschlagt bzw. komprimiert und nach unten geschoben, so dass sich die Federarme 117 am Boden des Aufnahmeraums 114 abstützen und elastisch verformen. Dies wird soweit erfolgen, bis eine Fluidverbindung zwischen dem Kanal 118 und einer Abrundung 119 im Bereich des Anschlusselementes vorliegt. Dann kann eine Fluidverbindung zwischen dem zu verbindenden Element und dem Aufnahmeraum 114 erfolgen. Statt der Abrundung können auch Nuten oder Kanäle in der Wandung des Anschlusselements vorgesehen sein, welche ab einer gewissen vorgebbaren Position des Ventilelements eine Fluidverbindung erlauben.

Wird das zu verbindende Element wieder aus dem Anschlusselement 107 entfernt, so entspannt sich das elastische formbare Ventilelement 115 wieder und der zylindrische Bereich 116 wird wieder nach oben geschoben, so dass das Anschlusselement 107 wieder abgedichtet ist.

Im unteren Bereich des Verbindungselements 101 ist radial innerhalb des zylindrischen Wandbereichs 112 ein Dorn 120 vorgesehen, welcher an seinem Ende ein verdicktes Element 121 trägt, in das bevorzugt ein Stift 122 des Dorns 120 eingesteckt ist oder bevorzugt einteilig mit dem Dorn 120 ausgebildet ist. Das Element 121 ist bevorzugt mittels Heißverprägen des Stifts befestigt.

Der Dorn 120 kann vorzugsweise mit einem Steg einteilig ausgebildet sein, welcher die Öffnungen 113 trennt.

Als Ventilelement ist ein hülsenförmiges Element 123 vorgesehen, welches in einen Boden 124 übergeht, wobei das hülsenförmige Element 123 etwa hohlzylindrisch ausgebildet ist und sich mit seinem axial unteren Randbereich an einer Schulter des Elements 121 abstützt. Zwischen dem Boden 124 und dem Boden 110 des Gehäuseteils ist ein Kraftspeicher 125, bevorzugt als ringförmiges elastisches Element, wie O-Ring, angeordnet, um eine Beaufschlagung des hülsenförmigen Elements 123 gegen das Element 121 zu beaufschlagen. Dadurch wird eine Abdichtung zwischen dem Aufnahmeraum 126 und dem Anschlusselement 127 erreicht, das durch das hülsenförmige Element 123 mit dem Boden 124 und der Umfangswandung 112 gebildet ist.

Wird von unten ein zu verbindendes Element mit einem umlaufenden Kragen in den Bereich 127 eingesteckt, so wird das hülsenförmige Element 123 mit dem Boden 124 axial nach oben geschoben, entgegen der Kraft des Kraftspeichers 125 und die Schulter 128 des hülsenförmigen Elements 123 hebt von einer Schulter des Elements 121 ab und erlaubt eine Fluidverbindung zwischen dem zu verbindenden Element durch den Kanal 129 im hülsenförmigen Element 123 bis zum Aufnahmeraum 126. Da zwischen dem Aufnahmeraum 126 und dem Aufnahmeraum 114 durch die Öffnungen 113 eine Fluidverbindung besteht, kann zwischen zwei zu verbindenden Elementen, die an den Anschlusselementen 107 und 127 angeschlossen sind, eine Fluidverbindung erzeugt werden.

Bevorzugt sind die elastischen Elemente 115 und 125 aus einem elastomeren Material und die Gehäuseelemente 103, 104 sowie das hülsenförmige Element 123 und das Element 121 aus einem nicht elastomeren, eher formstabilen, Material, wie aus einem Thermoplast. Dabei kann das Element 115 und das Element 125 bevorzugt aus Liquid Silicone Rubber (LSR) oder Hochtemperatur vernetzendem Silikonkautschuk (HTV) oder Raumtemperatur vernetzendem Silikonkautschuk (RTV) bestehen und die Gehäuseteile sowie die Hülse und das Abschlusselement 121 können aus Acrylnitril/Butadien/Styrol (ABS), Polycarbonat (PC), Polypropylen (PP) oder Polyethylen (PE) o.ä. bestehen.

Die Figur 7 zeigt die Ansicht des Verbindungselements 101, wobei die Figur 8 zeigt, dass der Aufnahmeraum 114 mit dem Deckel 105 im Schnitt rund ausgebildet ist.

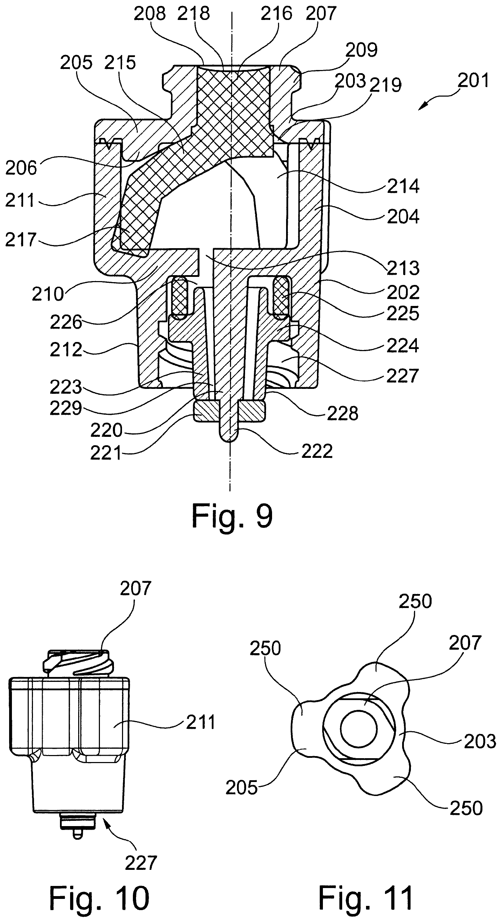

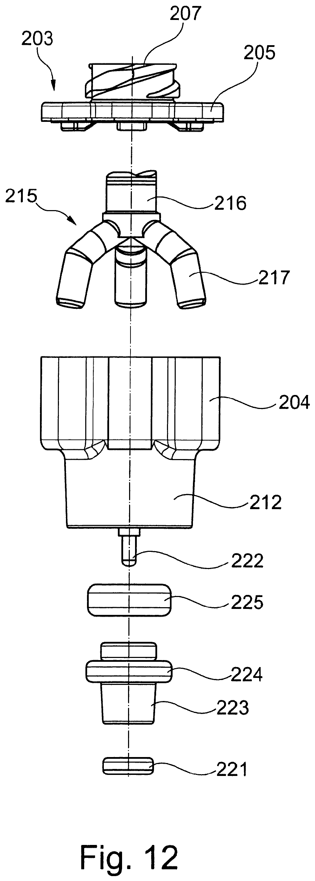

Die Figuren 9, 10, 11 und 12 zeigen ein weiteres Ausführungsbeispiel eines erfindungsgemäßen Verbindungselements 201 jeweils im Schnitt bzw. in einer Seitenansicht bzw. in einer Ansicht von oben.

Das Verbindungselement 201 ist mit einem Gehäuse 202 ausgebildet. Das Gehäuse 202 besteht aus zumindest zwei Gehäuseteilen 203,204. Im Ausführungsbeispiel der Figur 9 besteht das Gehäuse 202 aus den beiden Gehäuseteilen 203, 204, die miteinander verbunden sind.

Das erste Gehäuseteil 203 weist einen Boden 205 mit einem davon abragenden Steg 206 auf. Der Boden 205 ist mit dem Anschlusselement 207 versehen. Dazu ist das Anschlusselement 207 als Anschlussstutzen an dem Boden 205 angebracht. Vorteilhaft ist es mit dem Boden einteilig verbunden. Dabei kann in die Öffnung 208 des Anschlusselements 207 ein zu verbindendes Element eingesteckt werden. Zur Fixierung ist radial außerhalb des Anschlussstutzens 207 in der Wand des Stutzens ein Gewinde 209 oder ähnliches vorgesehen, mittels welchem das zu verbindende Element auch mit dem Anschlussstutzen 207 verschraubt oder verbunden, wie aufgesteckt, werden kann.

Das zweite Gehäuseteil 204 ist mit einem Boden 210 ausgebildet, von dem sich in eine Richtung eine nach oben abragende umlaufende Wand 211 erstreckt. In die entgegengesetzte Richtung ist eine umlaufende Wand 212 vorgesehen, die ebenso von dem Boden 210 abragt.

Der Boden 210 weist zumindest eine Öffnung oder mehrere Öffnungen 213 auf, um eine Fluidkommunikation zwischen den beiden Seiten des Bodens 210 zu erlauben. Dabei ist im Ausführungsbeispiel eine Mehrzahl von Öffnungen 213 vorgesehen, wobei auch nur eine Öffnung vorgesehen sein könnte. Vorzugsweise sind vier Öffnungen 213 vorgesehen. Vorteilhaft ist eine Anzahl von vier Öffnungen 213 vorgesehen, die durch einen kreuzförmigen Steg getrennt sind.

Wie in der Figur 9 zu erkennen ist, sind die beiden Gehäuseteile 203,204 im Bereich der umlaufenden Wand 211 und des Bades 205 miteinander verbunden, wie vorzugsweise verschweißt.

Das Gehäuse bildet einen ersten Aufnahmeraum 214 im Bereich der Verbindung der beiden Gehäuseteile 203,204, wobei der Aufnahmeraum 214 gebildet wird durch eine umlaufende Wand 211, einen Boden 205 des ersten Gehäuseteils und einen Boden 210 des zweiten Gehäuseteils.

Nach oben am Boden 205 des ersten Gehäuseteils 203 ist das Anschlusselement 207 vorgesehen.

Das Ventilelement 215 ist in dem Aufnahmeraum 214 angeordnet und weist einen zylindrischen Bereich 216 oder auch einen zylindrischen Körper 116 auf, von dem nach unten Federarme 217 abragen. Dabei ist der zylindrische Bereich 216 mit den Federarmen 217 vorteilhaft einteilig ausgebildet und die Federarme 217 ragen in einem Winkel zur Vertikalen nach radial außen und unten von dem zylindrischen Bereich 216 ab. Das elastische Federelement wird in den Figuren 21 bis 24 näher erläutert. Die Federarme 217 sind in dem Ausführungsbeispiel der Figuren 9 bis 11 abgewinkelt bzw. weisen Knicke auf. Die Federarme 217 bilden bei Vorhandensein von drei Federarmen 217 eine Anordnung annähernd einer Kantenanordnung eines Tetraeders.

In der in Figur 9 gezeigten Position des Ventilelements 215 ist eine Fluidverbindung zwischen dem Anschlusselement 207 und dem Aufnahmeraum 214 abgeschlossen und abgedichtet. Wird von oben in das Anschlusselement 207 ein Element eingesteckt, so wird der zylindrische Bereich 216 des Ventilelements 215 nach unten beaufschlagt bzw. komprimiert und nach unten geschoben, so dass sich die Federarme 217 am Boden des Aufnahmeraums 214 abstützen und elastisch verformen. Dies wird soweit erfolgen, bis eine Fluidverbindung zwischen dem Kanal 218 und einer Abrundung 219 im Bereich des Anschlusselementes vorliegt. Dann kann eine Fluidverbindung zwischen dem zu verbindenden Element und dem Aufnahmeraum 214 erfolgen. Statt der Abrundung können auch Nuten oder Kanäle in der Wandung des Anschlusselements vorgesehen sein, welche ab einer gewissen vorgebbaren Position des Ventilelements eine Fluidverbindung erlauben.

Wird das zu verbindende Element wieder aus dem Anschlusselement 207 entfernt, so entspannt sich das elastische formbare Ventilelement 215 wieder und der zylindrische Bereich 216 wird wieder nach oben geschoben, so dass das Anschlusselement 207 wieder abgedichtet ist.

Im unteren Bereich des Verbindungselements 201 ist radial innerhalb des zylindrischen Wandbereichs 212 ein Dorn 220 vorgesehen, welcher an seinem Ende ein verdicktes Element 221 trägt, in das bevorzugt ein Stift 222 des Dorns 220 eingesteckt ist oder bevorzugt einteilig mit dem Dorn 220 ausgebildet ist. Das Element 221 ist bevorzugt mittels Heißverprägen des Stifts befestigt.

Der Dorn 220 kann vorzugsweise mit einem Steg einteilig ausgebildet sein, welcher die Öffnungen 213 trennt.

Als Ventilelement ist ein hülsenförmiges Element 223 vorgesehen, welches in einen Flansch 224 übergeht, wobei das hülsenförmige Element 223 etwa hohlzylindrisch ausgebildet ist und sich mit seinem axial unteren Randbereich an einer Schulter des Elements 221 abstützt. Zwischen dem Flansch 224 und dem Boden 210 des Gehäuseteils ist ein Kraftspeicher 225, bevorzugt als ringförmiges elastisches Element, wie O-Ring, angeordnet, um eine Beaufschlagung des hülsenförmigen Elements 223 gegen das Element 221 zu beaufschlagen. Dadurch wird eine Abdichtung zwischen dem Aufnahmeraum 226 und dem Anschlusselement 227 erreicht, das durch das hülsenförmige Element 223 mit dem Boden 224 und der Umfangswandung 212 gebildet ist.

Wird von unten ein zu verbindendes Element mit einem umlaufenden Kragen in den Bereich 227 eingesteckt, so wird das hülsenförmige Element 223 mit dem Flansch 224 axial nach oben geschoben, entgegen der Kraft des Kraftspeichers 225 und die Schulter 228 des hülsenförmigen Elements 223 hebt von einer Schulter des Elements 221 ab und erlaubt eine Fluidverbindung zwischen dem zu verbindenden Element durch den Kanal 229 im hülsenförmigen Element 223 bis zum Aufnahmeraum 226. Da zwischen dem Aufnahmeraum 226 und dem Aufnahmeraum 214 durch die Öffnungen 213 eine Fluidverbindung besteht, kann zwischen zwei zu verbindenden Elementen, die an den Anschlusselementen 207 und 227 angeschlossen sind, eine Fluidverbindung erzeugt werden.

Bevorzugt sind die elastischen Elemente 215 und 225 aus einem elastomeren Material und die Gehäuseelemente 203, 204 sowie das hülsenförmige 223 und das Element 221 aus einem nicht elastomeren, eher formstabilen, Material, wie aus einem Thermoplast. Dabei kann das Element 215 und das Element 225 bevorzugt aus Liquid Silicone Rubber (LSR) oder Hochtemperatur vernetzendem Silikonkautschuk (HTV) oder Raumtemperatur vernetzendem Silikonkautschuk (RTV) bestehen und die Gehäuseteile sowie die Hülse und das Abschlusselement 221 können aus Acrylnitril/Butadien/Styrol (ABS), Polycarbonat (PC), Polypropylen (PP) oder Polyethylen (PE) o.ä. bestehen.

Die Figur 10 zeigt die Ansicht des Verbindungselements 201, wobei die Figur 11 zeigt, dass der Aufnahmeraum 214 mit dem Deckel 205 im Schnitt rund mit nach außen ragenden Taschen 250 ausgebildet ist. Dies bedeutet, dass der Querschnitt im Bereich der Taschen einen größeren Durchmesser hat und zwischen den Taschen im Durchmesser reduziert ist. Dies erbringt ein kleineres Volumen im Aufnahmeraum 214.

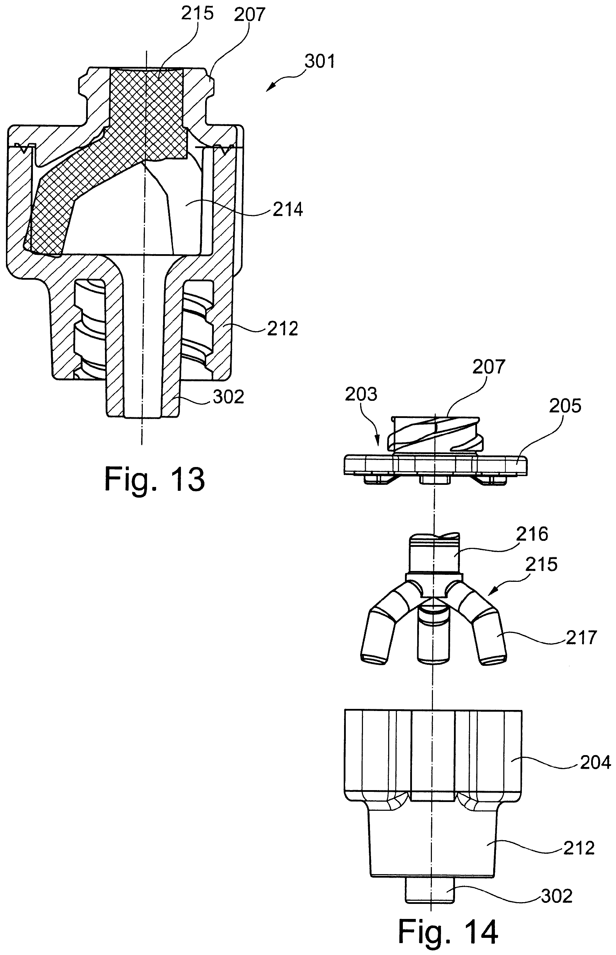

Die Figuren 13 und 14 zeigen ein weiteres Ausführungsbeispiel eines erfindungsgemäßen Verbindungselements 301 jeweils im Schnitt bzw. in einer Explosionsdarstellung.

Es ist zu erkennen, dass sich das Verbindungselement 301 vom Verbindungselement 201 dadurch unterscheidet, dass die Abdichteinheit mit Dorn 220, Hülse 223 und Federring 225 nicht vorgesehen ist, sondern lediglich ein rohrförmiger Stutzen 302.

Die verbleibenden identischen Bauteile werden bereits zu den Figuren 6 bis 11 beschrieben, so dass auf diese Beschreibung verwiesen wird.

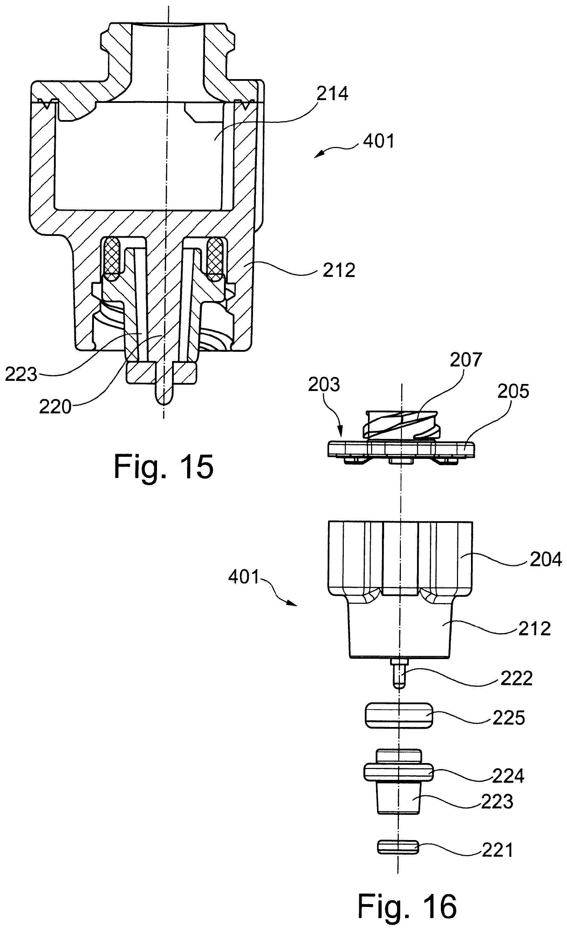

Die Figuren 15 und 16 zeigen ein weiteres Ausführungsbeispiel eines erfindungsgemäßen Verbindungselements 401 jeweils im Schnitt bzw. in einer Explosionsdarstellung.

Es ist zu erkennen, dass sich das Verbindungselement 401 vom Verbindungselement 201 dadurch unterscheidet, dass die Abdichteinheit mit dem Federelement 215 nicht vorgesehen ist.

Die verbleibenden identischen Bauteile werden bereits zu den Figuren 6 bis 11 beschrieben, so dass auf diese Beschreibung verwiesen wird.

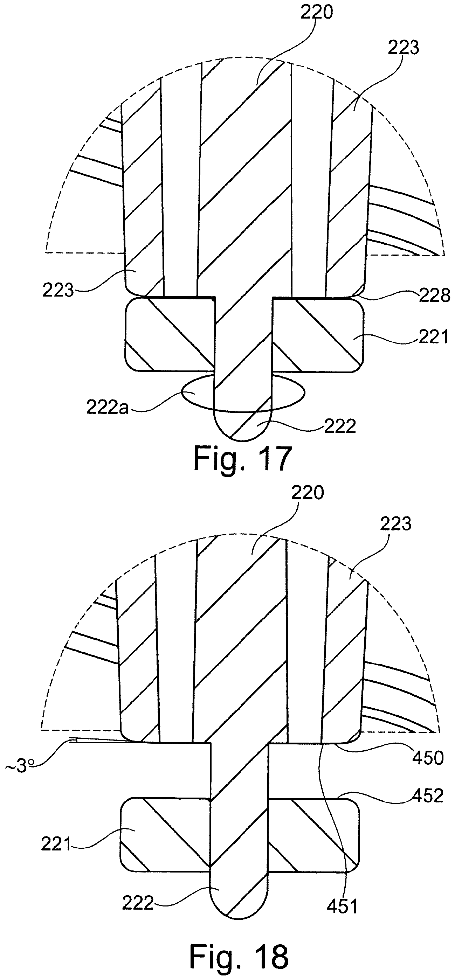

Die Figuren 17 und 18 zeigen die Abdichtung der Elemente123 und 121 bzw. 223 und 221 gegeneinander. Dabei ist das Element 221 mit dem Dorn 220 verbunden, wie beispielsweise heißverprägt. Dazu wird der überstehende Teil des Fingers 222 heißverprägt, so dass er eine Art Linse 222a bildet.

Die Unterkante 450 des Elements 223 weist einen Winkel von etwa 2° bis 4°, vorzugsweise 3° zur Horizontalen auf, so dass das Element 223 sich zuerst mit seinem inneren Rand 451 an die Oberfläche 452 des Elements 221 anlegt.

In Figur 17 ist die Fluidverbindung geschlossen bzw. unterbrochen und in Figur 18 ist die Fluidverbindung zugelassen, weil das Element 223 von dem Element 221 abgehoben ist.

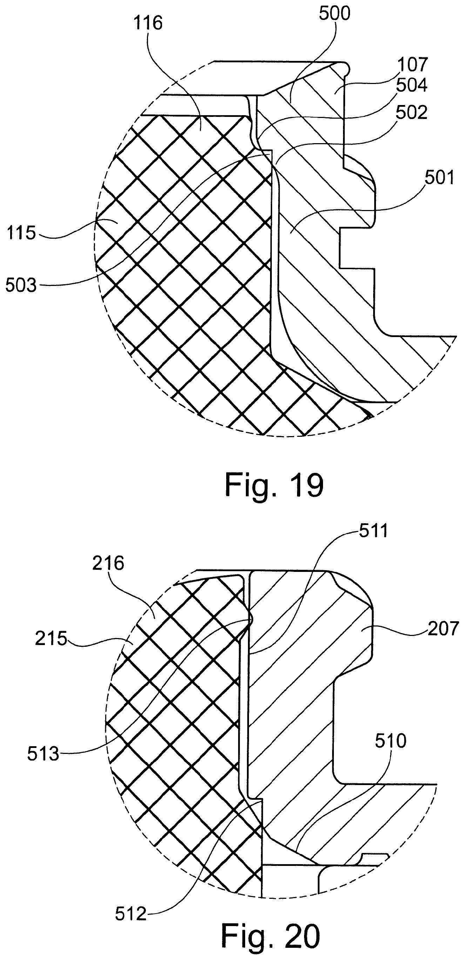

Die Figuren 19 und 20 zeigen zwei Ausführungsformen der Abdichtung zwischen dem Anschlusselement 107 bzw. 207 und dem zylindrischen Bereich 116 bzw. 216 des Federelements 115 bzw. 215.

In Figur 19 ist der zylindrische Bereich 116 des elastischen Federelements 115 in dem Anschlusselement 107 ausschnittweise gezeigt. Das Anschlusselement 107 weist an seinem oberen Endbereich 500 eine Durchmesserverjüngung auf, so dass der Durchmesser im oberen Endbereich 500 geringer ist als im darunter liegenden Bereich 501. Der Übergang 504 erfolgt mit einem vorgesehenen Radius bei 502. Das elastische Element 115 weist an seinem zylindrischen Bereich 116 ebenso eine Radiusveränderung auf, wobei der Radius im oberen Bereich kleiner ist als in einem unteren Bereich. Dabei wird der Radiusunterschied durch einen Absatz gebildet, der im Wesentlichen als rechtwinklige Stufe 503 betrachtet werden kann.

Ist der zylindrische Bereich 116 des elastischen Elements 115 in seiner oberen Stellung, so stößt die Stufe 503 an den Übergang 504 an und legt sich dichtend an den Übergang 504 an.

Die Figur 20 zeigt ein im Wesentlichen innen rohrförmiges Anschlusselement 207 mit einem im Wesentlichen geraden rohrförmigen Bereich 511, der im unteren Bereich in einen aufgeweiteten Bereich 510 übergeht.

Das elastische Federelement 215 weist an seinem zylindrischen Bereich 216 im oberen Bereich eine in radialer Richtung abstehende Dichtlippe 513 und im unteren Bereich eine Stufe 512 zur Durchmesservergrößerung auf.

In einer Position des zylindrischen Bereichs 216 in seiner oberen Stellung legt sich die Dichtlippe 513 an dem rohrförmigen Anschlusselement 207 dichtend an und die Stufe 512 legt sich in dem aufgeweiteten Bereich 510 des Anschlusselements 207 abdichtend an.

Die Figuren 21 bis 24 zeigen das elastische Federelement in verschiedenen Ansichten. In der Ansicht von oben gemäß Figur 21 ist der zylindrische Bereich 216 zu erkennen, welcher auf seiner Oberseite eine Struktur aufweist, die einen dreifachen Durchgang bzw. einen Durchgang mit drei Vertiefungen 530 zeigt, die etwa sternförmig zusammenlaufen. Die Vertiefungen 530 sind jeweils durch Erhöhungen 531 voneinander getrennt.

Von dem zylindrischen Bereich 216 ragen drei Federarme 217 ab, wobei die Federarme 217 einen Winkel von 120° zueinander einschließen.

Sie liegen bevorzugt in einer Ebene senkrecht zur Längsachse des zylindrischen Bereichs 216.

Die abragenden Federarme 217 sind vorteilhaft als gerade oder einfach oder mehrfach abgeknickte oder gekrümmte Federarme 217 ausgebildet. Im Ausführungsbeispiel der Figuren 21 bis 24 sind die Federarme 217 abgeknickt ausgebildet.

Dazu weisen die Federarme 217 einen ersten Bereich 533 auf, welcher in einem Winkel α1 von etwa 25 bis 30°, vorzugsweise 27°, zur Ebene senkrecht zur Längsachse des zylindrischen Bereichs 216 angeordnet ist.

Weiterhin weisen die Federarme 217 einen zweiten Bereich 534 auf, welcher in einem Winkel α2 von etwa 35 bis 40°, vorzugsweise 37°, zur Ebene senkrecht zur Längsachse des zylindrischen Bereichs 216 angeordnet ist.

Auch weisen die Federarme einen dritten Bereich auf, welcher in einem Winkel α3 von etwa 75 bis 80°, vorzugsweise 77°, zur Ebene senkrecht zur Längsachse des zylindrischen Bereichs 216 angeordnet ist.

Der erste Bereich 533 schließt sich dabei an den zylindrischen Bereich an. An diesen schließt sich der Bereich 534 an und an diesen der Bereich 535.

Die Figur 23 zeigt die Oberfläche 550 des zylindrischen Bereichs 216 in einer Seitenansicht. Man erkennt die Struktur der Oberfläche 550 mit den Vertiefungen 530 und den Erhöhungen 531.

Der Querschnitt der Federarme ist oval gemäß Figur 24. Dabei weist die Achse 540 in der kurzen Richtung auf den zylindrischen Bereich 216 hin und die Achse 541 der langen Richtung steht senkrecht dazu. Dies bewirkt, dass die Federarme 216 in Bewegungsrichtung der Federarme 217 leichter zu verformen sind als senkrecht dazu, was die Seitenstabilität gegen Verdrehungen erhöht. Alternativ kann der Querschnitt auch eine runde oder anderweitige Form aufweisen.

Im Hinblick auf die Querschnitte der Federarme können diese jeweils gleich ausgebildet sein. Auch kann einer der Federarme gegenüber den anderen Federarmen unterschiedlich ausgebildet sein, wie insbesondere mit einem größeren Querschnitt oder mit einem kleineren Querschnitt. Alternativ dazu können auch alle Federarme unterschiedlich ausgebildet sein, wie mit einem größeren oder kleineren Querschnitt versehen sein.

Dies bewirkt, dass bei Beaufschlagung des zylindrischen Bereichs von oben von der Richtung der Einlassöffnung durch das Anschlusselement, der zylindrische Bereich sich in seiner Endstellung seitlich zumindest geringfügig verkippen kann. Dies hat den Vorteil, dass die Stirnfläche des zylindrischen Elements auch bei glatter Ausführung ohne Kanäle eine gute Durchströmung des Verbindungselements erlaubt.

Offenbart wird weiterhin auch ein Ausführungsbeispiel, welches in Abwandlung des Ausführungsbeispiels der Figuren 1 oder 2 am Boden 10 gespiegelt ist und den oberen Teil der Vorrichtung bzw. den unteren Teil der Vorrichtung doppelt aufweist.

Gleiches gilt für ein Ausführungsbeispiel, welches der Ausführungsformen der Figur 6 im Wesentlichen entspricht, jedoch mit einer Spiegelung hinsichtlich dem Boden 110, so dass der obere Teil oder der untere Teil dupliziert ist.

Gleiches gilt für ein Ausführungsbeispiel, welches der Ausführungsformen der Figur 9 im Wesentlichen entspricht, jedoch mit einer Spiegelung hinsichtlich dem Boden 210, so dass der obere Teil oder der untere Teil dupliziert ist.

## Patentansprüche

1. Verbindungselement (1, 101, 201, 301, 401) zur Erzeugung einer Fluidverbindung zwischen zwei zu verbindenden Elementen, mit einem Gehäuse (2, 102, 202) mit einem ersten Aufnahmeraum (14, 114, 214) mit einem ersten Anschlusselement (7, 107, 207) und einem darin angeordneten ersten verlagerbaren Ventilelement (15, 115, 215) und mit einem zweiten Aufnahmeraum (26, 126, 226) mit einem zweiten Anschlusselement (27, 127, 227) mit einem darin angeordneten zweiten verlagerbaren Ventilelement (23, 123, 223), der erste und der zweite Aufnahmeraum (14, 114, 214, 26, 126, 226) sind miteinander fluidverbunden, wobei das erste Ventilelement (15, 115, 215) entgegen der Kraft eines ersten Kraftspeichers verlagerbar ist, wenn eines der zu verbindenden Elemente in das erste Anschlusselement eingesteckt wird, und das zweite Ventilelement (23, 123, 223) entgegen eines zweiten Kraftspeichers verlagerbar ist, wenn das zweite der zu verbindenden Elemente in das zweite Anschlusselement eingesteckt wird, wobei das erste Ventilelement (15, 115, 215) zwischen zwei Positionen verlagerbar ist, wobei in der ersten Position eine Fluidverbindung zwischen dem ersten Aufnahmeraum (14, 114, 214) und dem ersten Anschlusselement (7, 107, 207) durch das erste Ventilelement (15, 115, 215) unterbrochen ist und in der zweiten Position eine Fluidverbindung zwischen dem ersten Aufnahmeraum (14, 114, 214) und dem ersten Anschlusselement (7, 107, 207) durch das erste Ventilelement (15, 115, 215) geöffnet ist und wobei das zweite Ventilelement (23, 123, 223) zwischen zwei Positionen verlagerbar ist, wobei in der ersten Position eine Fluidverbindung zwischen dem zweiten Aufnahmeraum (26, 126, 226) und dem zweiten Anschlusselement (27, 127, 227) durch das zweite Ventilelement (23, 123, 223) unterbrochen ist und in der zweiten Position eine Fluidverbindung zwischen dem zweiten Aufnahmeraum (26, 126, 226) und dem zweiten Anschlusselement (27, 127, 227) durch das zweite Ventilelement (23, 123, 223) geöffnet ist, wobei das erste Anschlusselement (7, 107, 207) ein hohlzylindrisches Element aufweist und das erste Ventilelement (15, 115, 215) zumindest einen zylindrischen Bereich (16, 116, 216) zum Abdichten des ersten Anschlusselements aufweist, welcher in dem hohlzylindrischen Element verlagerbar aufgenommen ist und wobei das erste Ventilelement (15, 115, 215) an dem zylindrischen Bereich (16, 116, 216) abragende Federarme (17, 117, 217) aufweist, so dass der zylindrische Bereich (16, 116, 216) aus der zweiten Position wieder in die erste Position geschoben wird, wenn das zu verbindende erste Element aus dem ersten Anschlusselement entfernt wird, und sich das elastisch verformbare erste Ventilelement entspannt.

2. Verbindungselement nach Anspruch 1, **dadurch gekennzeichnet, dass** das Gehäuse (2, 102, 202) zumindest zweiteilig ausgebildet ist, wobei die zwei Gehäuseteile (3, 103, 203, 4, 104, 204) vorteilhaft miteinander verbunden sind, wie insbesondere nicht zerstörungsfrei lösbar miteinander verbunden sind.

3. Verbindungselement nach Anspruch 1 oder 2, **dadurch gekennzeichnet, dass** das Gehäuse (2, 102, 202) einteilig ausgebildet ist, wobei das Gehäuse (2, 102, 202) aus mehreren Gehäuseteilen (3, 103, 203, 4, 104, 204) bestehen kann, die miteinander verbunden, wie insbesondere nicht zerstörungsfrei lösbar miteinander verbunden sind.

4. Verbindungselement nach Anspruch 1 bis 3, **dadurch gekennzeichnet, dass** der erste Aufnahmeraum (14, 114, 214) von einem ersten und einem zweiten Gehäuseteil (3, 103, 203, 4, 104, 204) gebildet wird und der zweite Aufnahmeraum (26, 126, 226) von dem zweiten Gehäuseteil (4, 104, 204) gebildet wird.

5. Verbindungselement nach einem der vorhergehenden Ansprüche, **dadurch gekennzeichnet, dass** das erste Anschlusselement (7, 107, 207) an dem ersten Gehäuseteil (3, 103, 203) angeordnet oder ausgebildet ist und das zweite Anschlusselement (27, 127, 227) an dem zweiten Gehäuseteil (4, 104, 204) angeordnet oder ausgebildet ist.

6. Verbindungselement nach zumindest einem der vorhergehenden Ansprüche, **dadurch gekennzeichnet, dass** der erste Aufnahmeraum (14, 114, 214) einen im Schnitt kreisförmigen Querschnitt aufweist und von einer umlaufenden Wand (11, 111, 211) begrenzt ist, wobei der Aufnahmeraum (14, 114, 214) von zwei etwa ebenen Wandbereichen (5, 105, 205, 10, 110, 210) begrenzt wird, wobei das erste Anschlusselement (7, 107, 207) an einer der beiden Wände (5, 105, 205) angeordnet oder aufgenommen ist und in der anderen Wand (10, 110, 210) eine Fluidverbindung zu dem zweiten Aufnahmeraum (26, 126, 226) vorgesehen ist.

7. Verbindungselement nach zumindest einem der vorhergehenden Ansprüche, **dadurch gekennzeichnet, dass** der erste Aufnahmeraum (14, 114, 214) einen im Schnitt kreisförmigen Querschnitt mit nach außen ragenden Taschen (250) aufweist und von einer im Radius veränderlichen umlaufenden Wand begrenzt ist, wobei der Aufnahmeraum (14, 114, 214) von zwei etwa ebenen Wandbereichen (5, 105, 205, 10, 110, 210) begrenzt wird, wobei das erste Anschlusselement (7, 107, 207) an einer der beiden Wände (5, 105, 205) angeordnet oder aufgenommen ist und in der anderen Wand (10, 110, 210) eine Fluidverbindung zu dem zweiten Aufnahmeraum (26, 126, 226) vorgesehen ist.

8. Verbindungselement nach Anspruch 7, **dadurch gekennzeichnet, dass** die Anzahl der Taschen (250) der Anzahl von Federarmen (17, 117, 217) des Ventilelements entspricht.

9. Verbindungselement nach einem der vorhergehenden Ansprüche, **dadurch gekennzeichnet, dass** in jeder Tasche (250) ein Federarm (17, 117, 217) des Ventilelements (15, 115, 215) angeordnet ist.

10. Verbindungselement nach einem der Ansprüche 1 bis 9, **dadurch gekennzeichnet, dass** die abragenden Federarme (17, 117, 217) als gerade oder einfach oder mehrfach abgeknickte oder gekrümmte Federarme ausgebildet sind.

11. Verbindungselement nach einem der vorhergehenden Ansprüche, **dadurch gekennzeichnet, dass** die Federarme (17, 117, 217) des ersten Ventilelements (15, 115, 215) in einem Winkel von 120° zueinander in der Ebene senkrecht zur Längsachse des zylindrischen Bereichs (16, 116, 216) angeordnet sind.

12. Verbindungselement nach einem der vorhergehenden Ansprüche, **dadurch gekennzeichnet, dass** die Federarme (17, 117, 217) einen ersten Bereich aufweisen, welcher in einem Winkel von etwa 25 bis 30°, vorzugsweise 27°, zur Ebene senkrecht zur Längsachse des zylindrischen Bereichs (16, 116, 216) angeordnet ist.

13. Verbindungselement nach einem der vorhergehenden Ansprüche, **dadurch gekennzeichnet, dass** die Federarme (17, 117, 217) einen zweiten Bereich aufweisen, welcher in einem Winkel von etwa 35 bis 40°, vorzugsweise 37°, zur Ebene senkrecht zur Längsachse des zylindrischen Bereichs (16, 116, 216) angeordnet ist.

14. Verbindungselement nach einem der vorhergehenden Ansprüche, **dadurch gekennzeichnet, dass** die Federarme (17, 117, 217) einen dritten Bereich aufweisen, welcher in einem Winkel von etwa 75 bis 80°, vorzugsweise 77°, zur Ebene senkrecht zur Längsachse des zylindrischen Bereichs (16, 116, 216) angeordnet ist.

15. Verbindungselement nach zumindest einem der vorhergehenden Ansprüche, **dadurch gekennzeichnet, dass** der zweite Aufnahmeraum (26, 126, 226) einen Bereich mit im Schnitt kreisförmigen Querschnitt aufweist, der von einer umlaufenden Wand (12, 112, 212) begrenzt ist, wobei der Aufnahmeraum von zwei etwa ebenen Wandbereichen (10, 110, 210, 24, 124, 224) begrenzt wird, wobei das zweite Anschlusselement (27, 127, 227) an einer der beiden Wände (24, 124, 224) angeordnet oder aufgenommen ist und in der anderen Wand (10, 110, 210) eine Fluidverbindung zu dem ersten Aufnahmeraum (14, 114, 214) vorgesehen ist.

16. Verbindungselement nach Anspruch 15, **dadurch gekennzeichnet, dass** von der anderen Wand (10, 110, 210) ein Dorn (20, 120, 220) abragt, welcher in das zweite Anschlusselement (27, 127, 227) eingreift.

17. Verbindungselement nach Anspruch 15 oder 16, **dadurch gekennzeichnet, dass** die eine Wand (24, 124, 224) mit dem Anschlusselement (27, 127, 227) als zweites Ventilelement (23, 123, 223) entgegen der Kraft eines Kraftspeichers (25, 125, 225) verlagerbar ist und dadurch eine Fluidverbindung zwischen dem Anschlusselement (27, 127, 227) und dem in das Anschlusselement hineinragenden Dorn freigebbar ist.

18. Verbindungselement nach Anspruch 17, **dadurch gekennzeichnet, dass** das zweite Ventilelement (23, 123, 223) als ein hülsenförmiges Element ausgebildet ist, welches in einen Boden (24) übergeht oder von welchem ein Flansch absteht.

19. Verbindungselement nach Anspruch 17 oder 18, **dadurch gekennzeichnet, dass** das hülsenförmige Element (23) sich mit einem axialen Endbereich an einer Schulter des Elements (21) abstützt.

20. Verbindungselement nach einem der vorhergehenden Ansprüche, **dadurch gekennzeichnet, dass** der zweite Kraftspeicher (25, 125, 225) ein elastisches Element ist, welches in dem zweiten Aufnahmeraum (26, 126, 226) angeordnet ist.

21. Verbindungselement nach einem der vorhergehenden Ansprüche, **dadurch gekennzeichnet, dass** der zweite Kraftspeicher (25, 125, 225) ein elastischer Ring ist, wie vorzugsweise ein elastischer O-Ring.

22. Verbindungselement nach einem der vorhergehenden Ansprüche, **dadurch gekennzeichnet, dass** als erstes Ventilelement ein elastisches Federelement (15) vorgesehen ist mit einem zylindrischen Körper (16) und von dem zylindrischen Körper (16) abragenden Federarmen (17), wobei das Federelement (15) mit seinem zylindrischen Körper (16) und den Federarmen (17) aus einem elastischen Material gefertigt ist.

23. Verbindungselement nach zumindest einem der vorhergehenden Ansprüche, **dadurch gekennzeichnet, dass** der Querschnitt eines Federarms (17) rund oder oval oder mehreckig ausgebildet ist.

24. Verbindungselement nach Anspruch 23, **dadurch gekennzeichnet, dass** der Querschnitt des Federarms (17) oval ist, wobei der Querschnitt in einer Ebene durch die Mittelsenkrechte des Federelements (15) einen kleineren Durchmesser aufweist als die dazu senkrechte Richtung.

25. Verbindungselement nach einem der vorhergehenden Ansprüche 22 bis 24, **dadurch gekennzeichnet, dass** der zylindrische Körper (16, 116, 216) an einem Endbereich benachbart zu dem Übergang zu den Federarmen (17) eine erste umlaufende Dichtlippe (512) aufweist.

26. Verbindungselement nach einem der vorhergehenden Ansprüche 22 bis 25, **dadurch gekennzeichnet, dass** der zylindrische Körper (16, 116, 216) an einem Endbereich gegenüber dem Übergang zu den Federarmen (17) eine zweite umlaufende Dichtlippe (513) aufweist.

27. Verbindungselement nach Anspruch 25 oder 26, **dadurch gekennzeichnet, dass** die erste Dichtlippe (512) als umlaufender Absatz ausgebildet ist.

28. Verbindungselement nach Anspruch 25, 26 oder 27, **dadurch gekennzeichnet, dass** die zweite Dichtlippe (513) als von der zylindrischen Wandung des zylindrischen Körpers (16, 116, 216) vorragende Lippe ausgebildet ist.

## Claims

1. A connecting element (1, 101, 201, 301, 401) for creating a fluid connection between two elements to be connected, with a housing (2, 102, 202) having a first receiving space (14, 114, 214) with a first connector element (7, 107, 207) and a first displaceable valve element (15, 115, 215) located therein, and having a second receiving space (26, 126, 226) with a second connector element (27, 127, 227) having a second displaceable valve element (23, 123, 223) located therein, the first and second receiving spaces (14, 114, 214, 26, 126, 226) are fluidically connected to one another, wherein the first valve element (15, 115, 215) is displaceable in opposition to the force of a first force accumulator when one of the elements to be connected is inserted into the first connector element, and the second valve element (23, 123, 223) is displaceable in opposition to a second force accumulator when the second one of the elements to be connected is inserted into the second connector element, wherein the first valve element (15, 115, 215) is displaceable between two positions, wherein a fluid connection between the first receiving space (14, 114, 214) and the first connector element (7, 107, 207) is interrupted by the first valve element (15, 115, 215) in the first position, and a fluid connection between the first receiving space (14, 114, 214) and the first connector element (7, 107, 207) is opened by the first valve element (15, 115, 215) in the second position, and wherein the second valve element (23, 123, 223) is displaceable between two positions, wherein a fluid connection between the second receiving space (26, 126, 226) and the second connector element (27, 127, 227) is interrupted by the second valve element (23, 123, 223) in the first position, and a fluid connection between the second receiving space (26, 126, 226) and the second connector element (27, 127, 227) is opened by the second valve element (23, 123, 223) in the second position, wherein the first connector element (7, 107, 207) has a hollow, cylindrical element and the first valve element (15, 115, 215) has at least one cylindrical region (16, 116, 216) for sealing the first connector element that is displaceably accommodated in the hollow, cylindrical element and wherein the first valve element (15, 115, 215) has resilient arms (17, 117, 217) projecting from the cylindrical region (16, 116, 216) so that the cylindrical region (16, 116, 216) is pushed into the first position again from the second position if the first element to be connected is removed from the first connector element and the elastically deformable valve element relaxes.

2. The connecting element according to claim 1, **characterised in that** the housing (2, 102, 202) is implemented in at least two-piece form, wherein the two housing parts (3, 103, 203, 4, 104, 204) are advantageously connected to one another, such as, in particular, are connected to one another so that they cannot be separated non-destructively.

3. The connecting element according to claim 1 or 2, **characterised in that** the housing (2, 102, 202) is implemented in one piece, wherein the housing (2, 102, 202) can be composed of multiple housing parts (3, 103, 203, 4, 104, 204) that are connected to one another, such as, in particular, are connected to one another so that they cannot be separated non-destructively.

4. The connecting element according to claim 1 through 3, **characterised in that** the first receiving space (14, 114, 214) is composed of a first and a second housing part (3, 103, 203, 4, 104, 204), and the second receiving space (26, 126, 226) is composed of the second housing part (4, 104, 204).

5. The connecting element according to one of the preceding claims, **characterised in that** the first connector element (7, 107, 207) is located or formed on the first housing part (3, 103, 203) and the second connector element (27, 127, 227) is located or formed on the second housing part (4, 104, 204).

6. The connecting element according to at least one of the preceding claims, **characterised in that** the first receiving space (14, 114, 214) has a circular cross-section and is delimited by a surrounding wall (11, 111, 211), wherein the receiving space (14, 114, 214) is delimited by two approximately flat wall regions (5, 105, 205, 10, 110, 210), wherein the first connector element (7, 107, 207) is located or accommodated on one of the two walls (5, 105, 205) and a fluid connection to the second receiving space (26, 126, 226) is provided in the other wall (10, 110, 210).

7. The connecting element according to at least one of the preceding claims, **characterised in that** the first receiving space (14, 114, 214) has a circular cross-section with outwardly projecting pockets (250) and is delimited by a surrounding wall with changeable radius, wherein the receiving space (14, 114, 214) is delimited by two approximately flat wall regions (5, 105, 205, 10, 110, 210), wherein the first connector element (7, 107, 207) is located or accommodated on one of the two walls (5, 105, 205) and a fluid connection to the second receiving space (26, 126, 226) is provided in the other wall (10, 110, 210).

8. The connecting element according to claim 7, **characterised in that** the number of pockets (250) corresponds to the number of resilient arms (17, 117, 217) of the valve element.

9. The connecting element according to one of the preceding claims, **characterised in that** a resilient arm (17, 117, 217) of the valve element (15, 115, 215) is located in each pocket (250).

10. The connecting element according to one of claims 1 to 9, **characterised in that** the projecting resilient arms (17, 117, 217) are implemented as straight resilient arms or as resilient arms with one or multiple bends or curves.

11. The connecting element according to one of the preceding claims, **characterised in that** the resilient arms (17, 117, 217) of the first valve element (15, 115, 215) are arranged at an angle of 120° with respect to one another in the plane perpendicular to the longitudinal axis of the cylindrical region (16, 116, 216).

12. The connecting element according to one of the preceding claims, **characterised in that** the resilient arms (17, 117, 217) have a first region that is arranged at an angle of approximately 25° to 30°, preferably 27°, to the plane perpendicular to the longitudinal axis of the cylindrical region (16, 116, 216).

13. The connecting element according to one of the preceding claims, **characterised in that** the resilient arms (17, 117, 217) have a second region that is arranged at an angle of approximately 35° to 40°, preferably 37°, to the plane perpendicular to the longitudinal axis of the cylindrical region (16, 116, 216).

14. The connecting element according to one of the preceding claims, **characterised in that** the resilient arms (17, 117, 217) have a third region that is arranged at an angle of approximately 75° to 80°, preferably 77°, to the plane perpendicular to the longitudinal axis of the cylindrical region (16, 116, 216).

15. The connecting element according to at least one of the preceding claims, **characterised in that** the second receiving space (26, 126, 226) has a region with a circular cross-section that is delimited by a surrounding wall (12, 112, 212), wherein the receiving space is delimited by two approximately flat wall regions (10, 110, 210, 24, 124, 224), wherein the second connector element (27, 127, 227) is located or accommodated on one of the two walls (24, 124, 224) and a fluid connection to the first receiving space (14, 114, 214) is provided in the other wall (10, 110, 210).

16. The connecting element according to claim 15, **characterised in that** a pin (20, 120, 220) that engages the second connector element (27, 127, 227) projects from the other wall (10, 110, 210).

17. The connecting element according to claim 15 or 16, **characterised in that** the wall (24, 124, 224) with the connector element (27, 127, 227) as second valve element (23, 123, 223) is displaceable in opposition to the force of a force accumulator (25, 125, 225), and a fluid connection between the connector element (27, 127, 227) and the pin projecting into the connector element can be opened by this means.

18. The connecting element according to claim 17, **characterised in that** the second valve element (23, 123, 223) is implemented as a sleeve-like element, which transitions into a floor (24), or from which a flange projects.

19. The connecting element according to claim 17 or 18, **characterised in that** an axial edge region of the sleeve-like element (23) braces against a shoulder of the element (21).

20. The connecting element according to one of the preceding claims, **characterised in that** the second force accumulator (25, 125, 225) is an elastic element that is located in the second receiving space (26, 126, 226).

21. The connecting element according to one of the preceding claims, **characterised in that** the second force accumulator (25, 125, 225) is an elastic ring, preferably such as an elastic O-ring.

22. The connecting element according to one of the preceding claims, **characterised in that** an elastic spring element (15) with a cylindrical body (16) and resilient arms (17) projecting from the cylindrical body (16) is provided as a first valve element, wherein the spring element (15) with its cylindrical body (16) and the resilient arms (17) is made of an elastic material.

23. The connecting element according to at least one of the preceding claims, **characterised in that** the cross-section of a resilient arm (17) is round or oval or polygonal.

24. The connecting element according to claim 23, **characterised in that** the cross-section of the resilient arm (17) is oval, wherein the cross-section in a plane through the mid-perpendicular of the spring element (15) has a smaller diameter than the direction perpendicular thereto.

25. The connecting element according to one of the preceding claims 22 to 24, **characterised in that** the cylindrical body (16, 116, 216) has a first circumferential sealing lip (512) at an end region adjacent to the transition to the resilient arms (17).

26. The connecting element according to one of the preceding claims 22 to 25, **characterised in that** the cylindrical body (16, 116, 216) has a second circumferential sealing lip (513) at an end region opposite the transition to the resilient arms (17).

27. The connecting element according to claim 25 or 26, **characterised in that** the first sealing lip (512) is implemented as a circumferential shoulder.

28. The connecting element according to claim 25, 26 or 27, **characterised in that** the second sealing lip (513) is implemented as a lip protruding from the cylindrical wall of the cylindrical body (16, 116, 216).

## Revendications

1. Elément de liaison (1, 101, 201, 301, 401) servant à la création d'une communication de fluide se produisant entre deux éléments à relier, ledit élément de liaison comprenant un boîtier (2, 102, 202) comportant un premier espace de logement (14, 114, 214) avec un premier élément de jonction (7, 107, 207) et un premier élément de soupape déplaçable (15, 115, 215) disposé dans ledit premier espace de logement, et comportant un deuxième espace de logement (26, 126, 226) avec un deuxième élément de jonction (27, 127, 227) comprenant un deuxième élément de soupape déplaçable (23, 123, 223) disposé dans ledit deuxième espace de logement, les premier et deuxième espaces de logement (14, 114, 214, 26, 126, 226) sont reliées fluidiquement l'un à l'autre, où le premier élément de soupape (15, 115, 215) est déplaçable en s'opposant à la force d'un premier dispositif accumulateur d'énergie, lorsque l'un des éléments à relier est inséré dans le premier élément de jonction, et le deuxième élément de soupape (23, 123, 223) est déplaçable en s'opposant à un deuxième dispositif accumulateur d'énergie, lorsque le deuxième des éléments à relier est inséré dans le deuxième élément de jonction, où le premier élément de soupape (15, 115, 215) est déplaçable entre deux positions, où, dans la première position, une communication de fluide se produisant entre le premier espace de logement (14, 114, 214) et le premier élément de jonction (7, 107, 207) est interrompue par le premier élément de soupape (15, 115, 215) et, dans la deuxième position, une communication de fluide se produisant entre le premier espace de logement (14, 114, 214) et le premier élément de jonction (7, 107, 207) est ouverte par le premier élément de soupape (15, 115, 215), et où le deuxième élément de soupape (23, 123, 223) est déplaçable entre deux positions, où, dans la première position, une communication de fluide se produisant entre le deuxième espace de logement (26, 126, 226) et le deuxième élément de jonction (27, 127, 227) est interrompue par le deuxième élément de soupape (23, 123, 223) et, dans la deuxième position, une communication de fluide se produisant entre le deuxième espace de logement (26, 126, 226) et le deuxième élément de jonction (27, 127, 227) est ouverte par le deuxième élément de soupape (23, 123, 223), où le premier élément de jonction (7, 107, 207) présente un élément cylindrique creux et le premier élément de soupape (15, 115, 215) présente au moins une zone cylindrique (16, 116, 216) servant à assurer l'étanchéité du premier élément de jonction, laquelle zone cylindrique est logée en étant déplaçable dans l'élément cylindrique creux, et où le premier élément de soupape (15, 115, 215) présente des bras à ressort (17, 117, 217) faisant saillie sur la zone cylindrique (16, 116, 216), de sorte que la zone cylindrique (16, 116, 216) est déplacée, passant à nouveau de la deuxième position dans la première position, lorsque le premier élément à relier est éloigné du premier élément de jonction et que le premier élément de soupape élastiquement déformable se détend.

2. Elément de liaison selon la revendication 1, **caractérisé en ce que** le boîtier (2, 102, 202) est configuré au moins en deux parties, où les deux parties de boîtier (3, 103, 203, 4, 104, 204) sont assemblées avantageusement l'une à l'autre, comme étant en particulier assemblées l'une à l'autre de manière non détachable et non destructive.

3. Elément de liaison selon la revendication 1 ou 2, **caractérisé en ce que** le boîtier (2, 102, 202) est configuré en une seule partie, où le boîtier (2, 102, 202) peut se composer de plusieurs parties de boîtier (3, 103, 203, 4, 104, 204), qui sont assemblées les unes aux autres, comme étant en particulier assemblées les unes aux autres de manière non détachable et non destructive.

4. Elément de liaison selon les revendications 1 à 3, **caractérisé en ce que** le premier espace de logement (14, 114, 214) est formé par une première et une deuxième partie de boîtier (3, 103, 203, 4, 104, 204), et le deuxième espace de logement (26, 126, 226) est formé par la deuxième partie de boîtier (4, 104, 204).

5. Elément de liaison selon l'une quelconque des revendications précédentes, **caractérisé en ce que** le premier élément de jonction (7, 107, 207) est disposé ou configuré sur la première partie de boîtier (3, 103, 203), et le deuxième élément de jonction (27, 127, 227) est disposé ou configuré sur la deuxième partie de boîtier (4, 104, 204).

6. Elément de liaison selon au moins l'une quelconque des revendications précédentes, **caractérisé en ce que** le premier espace de logement (14, 114, 214) présente en coupe une section de forme circulaire et est délimité par une paroi périphérique (11, 111, 211), où l'espace de logement (14, 114, 214) est délimité par deux zones de paroi (5, 105, 205, 10, 110, 210) à peu près plates, où le premier élément de jonction (7, 107, 207) est disposé ou logé sur l'une des deux parois (5, 105, 205) et, dans l'autre paroi (10, 110, 210), il est prévu une communication de fluide avec le deuxième espace de logement (26, 126, 226).

7. Elément de liaison selon au moins l'une quelconque des revendications précédentes, **caractérisé en ce que** le premier espace de logement (14, 114, 214) présente en coupe une section de forme circulaire, avec des poches (250) faisant saillie vers l'extérieur, et est délimité par une paroi périphérique variable en rayon, où l'espace de logement (14, 114, 214) est délimité par deux zones de paroi (5, 105, 205, 10, 110, 210) à peu près plates, où le premier élément de jonction (7, 107, 207) est disposé ou logé sur l'une des deux parois (5, 105, 205) et, dans l'autre paroi (10, 110, 210), il est prévu une communication de fluide avec le deuxième espace de logement (26, 126, 226).

8. Elément de liaison selon la revendication 7, **caractérisé en ce que** le nombre de poches (250) correspond au nombre de bras à ressort (17, 117, 217) de l'élément de soupape.

9. Elément de liaison selon l'une quelconque des revendications précédentes, **caractérisé en ce qu'**un bras à ressort (17, 117, 217) de l'élément de soupape (15, 115, 215) est disposé dans chaque poche (250).

10. Elément de liaison selon l'une quelconque des revendications 1 à 9, **caractérisé en ce que** les bras à ressort (17, 117, 217) faisant saillie sont configurés comme des bras à ressort droits ou bien coudés ou courbés une fois ou plusieurs fois.

11. Elément de liaison selon l'une quelconque des revendications précédentes, **caractérisé en ce que** les bras à ressort (17, 117, 217) du premier élément de soupape (15, 115, 215) sont disposés suivant un angle de 120° l'un par rapport à l'autre dans le plan perpendiculaire à l'axe longitudinal de la zone cylindrique (16, 116, 216).

12. Elément de liaison selon l'une quelconque des revendications précédentes, **caractérisé en ce que** les bras à ressort (17, 117, 217) présentent une première zone qui est disposée suivant un angle d'à peu près 25° à 30°, de préférence de 27°, par rapport au plan perpendiculaire à l'axe longitudinal de la zone cylindrique (16, 116, 216).

13. Elément de liaison selon l'une quelconque des revendications précédentes, **caractérisé en ce que** les bras à ressort (17, 117, 217) présentent une deuxième zone qui est disposée suivant un angle d'à peu près 35° à 40°, de préférence de 37°, par rapport au plan perpendiculaire à l'axe longitudinal de la zone cylindrique (16, 116, 216).

14. Elément de liaison selon l'une quelconque des revendications précédentes, **caractérisé en ce que** les bras à ressort (17, 117, 217) présentent une troisième zone qui est disposée suivant un angle d'à peu près 75° à 80°, de préférence de 77°, par rapport au plan perpendiculaire à l'axe longitudinal de la zone cylindrique (16, 116, 216).

15. Elément de liaison selon au moins l'une quelconque des revendications précédentes, **caractérisé en ce que** le deuxième espace de logement (26, 126, 226) présente une zone ayant en coupe une section de forme circulaire, ledit deuxième espace de logement étant délimité par une paroi périphérique (12, 112, 212), où l'espace de logement est délimité par deux zones de paroi (10, 110, 210, 24, 124, 224) à peu près plates, où le deuxième élément de jonction (27, 127, 227) est disposé ou logé sur l'une des deux parois (24, 124, 224), et il est prévu, dans l'autre paroi (10, 110, 210), une communication de fluide avec le premier espace de logement (14, 114, 214).

16. Elément de liaison selon la revendication 15, **caractérisé en ce qu'**un mandrin (20, 120, 220) dépasse de l'autre paroi (10, 110, 210), lequel mandrin s'engage dans le deuxième élément de jonction (27, 127, 227).

17. Elément de liaison selon la revendication 15 ou 16, **caractérisé en ce qu'**une des parois (24, 124, 224), avec le deuxième élément de jonction (27, 127, 227), est déplaçable en tant que deuxième élément de soupape (23, 123, 223), en s'opposant à la force d'un dispositif accumulateur d'énergie (25, 125, 225), et il en résulte qu'une communication de fluide, qui se produit entre l'élément de jonction (27, 127, 227) et le mandrin s'engageant dans l'élément de jonction, peut être libérée.

18. Elément de liaison selon la revendication 17, **caractérisé en ce que** le deuxième élément de soupape (23, 123, 223) est configuré comme un élément en forme de manchon, lequel élément passe dans un fond (24), ou bien fond par rapport auquel une bride fait saillie.

19. Elément de liaison selon la revendication 17 ou 18, **caractérisé en ce que** l'élément en forme de manchon (23) vient en appui, par une zone d'extrémité axiale, sur un épaulement de l'élément (21).

20. Elément de liaison selon l'une quelconque des revendications précédentes, **caractérisé en ce que** le deuxième dispositif accumulateur d'énergie (25, 125, 225) est un élément élastique qui est disposé dans le deuxième espace de logement (26, 126, 226).

21. Elément de liaison selon l'une quelconque des revendications précédentes, **caractérisé en ce que** le deuxième dispositif accumulateur d'énergie (25, 125, 225) est un anneau élastique, comme de préférence un anneau torique élastique.

22. Elément de liaison selon l'une quelconque des revendications précédentes, **caractérisé en ce qu'**il est prévu, comme premier élément de soupape, un élément à ressort élastique (15) avec un corps cylindrique (16) et des bras à ressort (17) faisant saillie par rapport au corps cylindrique (16), où l'élément à ressort (15), avec son corps cylindrique (16) et les bras à ressort (17), est fabriqué dans un matériau élastique.

23. Elément de liaison selon au moins l'une quelconque des revendications précédentes, **caractérisé en ce que** la section d'un bras à ressort (17) est ronde ou ovale ou polygonale.

24. Elément de liaison selon la revendication 23, **caractérisé en ce que** la section du bras à ressort (17) est ovale, où la section, dans un plan passant par la médiatrice de l'élément à ressort (15), présente un plus petit diamètre que la direction perpendiculaire à ladite médiatrice.

25. Elément de liaison selon l'une quelconque des revendications 22 à 24, **caractérisé en ce que** le corps cylindrique (16, 116, 216) présente une première lèvre d'étanchéité périphérique (512) au niveau d'une zone d'extrémité adjacente à la transition vers les bras à ressort (17).

26. Elément de liaison selon l'une quelconque des revendications 22 à 25, **caractérisé en ce que** le corps cylindrique (16, 116, 216) présente une deuxième lèvre d'étanchéité périphérique (513) au niveau d'une zone d'extrémité faisant face à la transition vers les bras à ressort (17).

27. Elément de liaison selon la revendication 25 ou 26, **caractérisé en ce que** la première lèvre d'étanchéité (512) est configurée comme un épaulement périphérique.

28. Elément de liaison selon la revendication 25, 26 ou 27, **caractérisé en ce que** la deuxième lèvre d'étanchéité (513) est configurée comme une lèvre faisant saillie par rapport à la paroi cylindrique du corps cylindrique (16, 116, 216).
